# EUROPEAN PATENT APPLICATION

(11) **EP 3 957 166 A1**
(43) Date of publication of application: **23.02.2022**
(21) Application number: 20790756.9
(22) Date of filing: 17.04.2020
(51) Int. Cl.: A01H 5/00, A01H 5/10, A01H 6/14, C12N 15/05

(54) **CYTOPLASMIC MALE STERILE PLANT OF GENUS LACTUCA HAVING IMPROVED LOW TEMPERATURE GROWTH ABILITY**

(30) Priority: 17.04.2019 JP 2019078905
(71) Applicant: Sakata Seed Corporation, Yokohama-shi Kanagawa 224-0041 (JP)
(72) Inventor: HORIUCHI, Shingo, Yokohama-shi, Kanagawa 224-0041 (JP); SUZUKI, Takao, Yokohama-shi, Kanagawa 224-0041 (JP); IZUMIDA, Atsushi, Yokohama-shi, Kanagawa 224-0041 (JP); TANAKA, Yasuo, Yokohama-shi, Kanagawa 224-0041 (JP)
(74) Representative: Bandpay & Greuter
(86) International application number: PCT/JP2020/016927
(87) International publication number: WO 2020/213727

(57) **Abstract**

The present specification discloses a cytoplasmic male sterile plant of the genus Lactuca having low temperature growth ability comparable to that of a plant of the genus Lactuca with a normal cytoplasm, or a progeny thereof. According to an embodiment of the present invention, it is possible to alleviate a reduction in the growth ability at low temperature observed in previous CMS plants of the genus Lactuca and provide a CMS plant of the genus Lactuca having low temperature growth ability.

SEQUENCE LISTING

<110>  Sakata Seed Corporation

<120>  CMS Lectuca species Plant modified in low temperature growth

<130>  800518JP01

<160>  90

<170>  PatentIn version 3.5

<210>  1
<211>  23
<212>  DNA
<213>  Artificial Sequence

<220>
<223>  primer

<400>  1
gctaactctc agtttggtcc tac                                               23


<210>  2
<211>  20
<212>  DNA
<213>  Artificial Sequence

<220>
<223>  primer

<400>  2
ccagaccggt taatgcaaga                                                   20


<210>  3
<211>  22
<212>  DNA
<213>  Artificial Sequence

<220>
<223>  primer

<400>  3
agccagtaac agacacgaca ga                                                22


<210>  4
<211>  22
<212>  DNA
<213>  Artificial Sequence

<220>
<223>  primer

<400>  4
cgaaccgtcc aatgaagaat aa                                                22


<210>  5
<211>  21
<212>  DNA

<213>  Artificial Sequence

<220>
<223>  primer

<400>  5
tctgtcgctt ttaaccactc g                                                21


<210>  6
<211>  22
<212>  DNA
<213>  Artificial Sequence

<220>
<223>  primer

<400>  6
ttcaaatcct acttggggag aa                                               22


<210>  7
<211>  21
<212>  DNA
<213>  Artificial Sequence

<220>
<223>  primer

<400>  7
tcaattaggg aatagcgcag a                                                21


<210>  8
<211>  21
<212>  DNA
<213>  Artificial Sequence

<220>
<223>  primer

<400>  8
tggtgtgcta accaaagaac c                                                21


<210>  9
<211>  21
<212>  DNA
<213>  Artificial Sequence

<220>
<223>  primer

<400>  9
agatcggatt tccttttcgt g                                                21


<210>  10
<211>  21
<212>  DNA
<213>  Artificial Sequence

<220>

<223>  primer

<400>  10
agcctatcgt ctcagccttt c                                                21


<210>  11
<211>  21
<212>  DNA
<213>  Artificial Sequence

<220>
<223>  primer

<400>  11
gcgctaggca cctcttactc t                                                21


<210>  12
<211>  21
<212>  DNA
<213>  Artificial Sequence

<220>
<223>  primer

<400>  12
ttgacagatt cctggtcgaa g                                                21


<210>  13
<211>  21
<212>  DNA
<213>  Artificial Sequence

<220>
<223>  primer

<400>  13
gacagcaaaa gcagcgatta g                                                21


<210>  14
<211>  21
<212>  DNA
<213>  Artificial Sequence

<220>
<223>  primer

<400>  14
atatgggaca agcggtaggt t                                                21


<210>  15
<211>  22
<212>  DNA
<213>  Artificial Sequence

<220>
<223>  primer

<400>  15

tgcaatgatg gagatggact ac                                              22

<210>  16
<211>  22
<212>  DNA
<213>  Artificial Sequence

<220>
<223>  primer

<400>  16
aacctccgaa acgataaaca ga                                              22

<210>  17
<211>  21
<212>  DNA
<213>  Artificial Sequence

<220>
<223>  primer

<400>  17
tggttctcat ttcctggttt g                                               21

<210>  18
<211>  21
<212>  DNA
<213>  Artificial Sequence

<220>
<223>  primer

<400>  18
caaagtgttc tcggactgca t                                               21

<210>  19
<211>  22
<212>  DNA
<213>  Artificial Sequence

<220>
<223>  primer

<400>  19
gtgaggctcc actctatcct gt                                              22

<210>  20
<211>  22
<212>  DNA
<213>  Artificial Sequence

<220>
<223>  primer

<400>  20
caatgctaaa tgtccaggga gt                                              22

<210>  21
<211>  22
<212>  DNA
<213>  Artificial Sequence

<220>
<223>  primer

<400>  21
gtactcaaca ggaagcgtca ca                                                22


<210>  22
<211>  21
<212>  DNA
<213>  Artificial Sequence

<220>
<223>  primer

<400>  22
cgaaagcgga tcagtacaaa c                                                 21


<210>  23
<211>  22
<212>  DNA
<213>  Artificial Sequence

<220>
<223>  primer

<400>  23
atcacgagtt tttctccatt cc                                                22


<210>  24
<211>  22
<212>  DNA
<213>  Artificial Sequence

<220>
<223>  primer

<400>  24
tttcccggta gaaccctatt tc                                                22


<210>  25
<211>  20
<212>  DNA
<213>  Artificial Sequence

<220>
<223>  primer

<400>  25
tagacagcaa gcaagcgaga                                                   20


<210>  26
<211>  20
<212>  DNA

<213>  Artificial Sequence

<220>
<223>  primer

<400>  26
gccctacctg atccatttca                                                   20


<210>  27
<211>  21
<212>  DNA
<213>  Artificial Sequence

<220>
<223>  primer

<400>  27
ctgccgatgt agctcttgtt c                                                 21


<210>  28
<211>  21
<212>  DNA
<213>  Artificial Sequence

<220>
<223>  primer

<400>  28
ggatgcacgt cttttgttca t                                                 21


<210>  29
<211>  20
<212>  DNA
<213>  Artificial Sequence

<220>
<223>  primer

<400>  29
caaaggcgag catatccatt                                                   20


<210>  30
<211>  21
<212>  DNA
<213>  Artificial Sequence

<220>
<223>  primer

<400>  30
tccatttccc tctcggtact t                                                 21


<210>  31
<211>  22
<212>  DNA
<213>  Artificial Sequence

<220>

<223>  primer

<400>  31
ccttgatcca cttggctaca tc                                              22


<210>  32
<211>  19
<212>  DNA
<213>  Artificial Sequence

<220>
<223>  primer

<400>  32
ggaatcgaac ccgtgtctt                                                  19


<210>  33
<211>  20
<212>  DNA
<213>  Artificial Sequence

<220>
<223>  primer

<400>  33
aaagagtcca gccgagcata                                                 20


<210>  34
<211>  21
<212>  DNA
<213>  Artificial Sequence

<220>
<223>  primer

<400>  34
aatcactatg gaaccggacc t                                               21


<210>  35
<211>  21
<212>  DNA
<213>  Artificial Sequence

<220>
<223>  primer

<400>  35
taagagcaaa caaggcaatg g                                               21


<210>  36
<211>  22
<212>  DNA
<213>  Artificial Sequence

<220>
<223>  primer

<400>  36

cggattcacc taaacatctt cc                                                22

<210>  37
<211>  22
<212>  DNA
<213>  Artificial Sequence

<220>
<223>  primer

<400>  37
tagcttggta atccctcgct tt                                                22

<210>  38
<211>  22
<212>  DNA
<213>  Artificial Sequence

<220>
<223>  primer

<400>  38
aagttgatga ttcccaccaa ga                                                22

<210>  39
<211>  21
<212>  DNA
<213>  Artificial Sequence

<220>
<223>  primer

<400>  39
aatcccagga gctaatccaa a                                                 21

<210>  40
<211>  22
<212>  DNA
<213>  Artificial Sequence

<220>
<223>  primer

<400>  40
atgatggcct ttctgatctc at                                                22

<210>  41
<211>  21
<212>  DNA
<213>  Artificial Sequence

<220>
<223>  primer

<400>  41
ccattgtagt tggagcgaga g                                                 21

<210>  42
<211>  21
<212>  DNA
<213>  Artificial Sequence

<220>
<223>  primer

<400>  42
ttccctgttc caaacatttc c                                                21


<210>  43
<211>  21
<212>  DNA
<213>  Artificial Sequence

<220>
<223>  primer

<400>  43
ttgttgagag gacttgcatc c                                                21


<210>  44
<211>  21
<212>  DNA
<213>  Artificial Sequence

<220>
<223>  primer

<400>  44
   cataccttgg cagtgtggtc t                                                  21


<210>  45
<211>  21
<212>  DNA
<213>  Artificial Sequence

<220>
<223>  primer

<400>  45
tcttggagac agggcaatag a                                                21


<210>  46
<211>  21
<212>  DNA
<213>  Artificial Sequence

<220>
<223>  primer

<400>  46
catcttaggc ggtaagcgaa g                                                21


<210>  47
<211>  21
<212>  DNA

<213>  Artificial Sequence

<220>
<223>  primer

<400>  47
gaaccgggcg tactacttct c                                                21


<210>  48
<211>  21
<212>  DNA
<213>  Artificial Sequence

<220>
<223>  primer

<400>  48
caaataaggt tacgggcttc c                                                21


<210>  49
<211>  22
<212>  DNA
<213>  Artificial Sequence

<220>
<223>  primer

<400>  49
taccctgtaa gcgtgaagtc ag                                               22


<210>  50
<211>  21
<212>  DNA
<213>  Artificial Sequence

<220>
<223>  primer

<400>  50
atgaaccact ccggtacaac a                                                21


<210>  51
<211>  21
<212>  DNA
<213>  Artificial Sequence

<220>
<223>  primer

<400>  51
agcggagcct agaagaagat g                                                21


<210>  52
<211>  20
<212>  DNA
<213>  Artificial Sequence

<220>

<223>  primer

<400>  52
tgaggaaggt acagcccaac                                                   20


<210>  53
<211>  21
<212>  DNA
<213>  Artificial Sequence

<220>
<223>  primer

<400>  53
gaaagcaact tcaccgaaag g                                                 21


<210>  54
<211>  21
<212>  DNA
<213>  Artificial Sequence

<220>
<223>  primer

<400>  54
atttcccacg tatggagcac t                                                 21


<210>  55
<211>  22
<212>  DNA
<213>  Artificial Sequence

<220>
<223>  primer

<400>  55
cgtacaacca tcaaaccaga ga                                                22


<210>  56
<211>  22
<212>  DNA
<213>  Artificial Sequence

<220>
<223>  primer

<400>  56
aacttgaagg tatggctgag tg                                                22


<210>  57
<211>  21
<212>  DNA
<213>  Artificial Sequence

<220>
<223>  primer

<400>  57

gtcgatcttg taacccacga a                                                21

<210>  58
<211>  21
<212>  DNA
<213>  Artificial Sequence

<220>
<223>  primer

<400>  58
gtcactcact ggaggtttgg a                                                21

<210>  59
<211>  21
<212>  DNA
<213>  Artificial Sequence

<220>
<223>  primer

<400>  59
tcaagtgaag tgcaagatcc a                                                21

<210>  60
<211>  21
<212>  DNA
<213>  Artificial Sequence

<220>
<223>  primer

<400>  60
gcgttttgtc ccctatctct c                                                21

<210>  61
<211>  21
<212>  DNA
<213>  Artificial Sequence

<220>
<223>  primer

<400>  61
ccaggggaca aatcaatagg a                                                21

<210>  62
<211>  21
<212>  DNA
<213>  Artificial Sequence

<220>
<223>  primer

<400>  62
gaataacatg gcctgaaacg a                                                21

<210>  63
<211>  21
<212>  DNA
<213>  Artificial Sequence

<220>
<223>  primer

<400>  63
gcaccaaacc aatccatctt c                                                21


<210>  64
<211>  21
<212>  DNA
<213>  Artificial Sequence

<220>
<223>  primer

<400>  64
tggcttcctc cctagctcta c                                                21


<210>  65
<211>  21
<212>  DNA
<213>  Artificial Sequence

<220>
<223>  primer

<400>  65
gagcttctcc caaggatgtc t                                                21


<210>  66
<211>  21
<212>  DNA
<213>  Artificial Sequence

<220>
<223>  primer

<400>  66
attctgtgat cgaatgccac t                                                21


<210>  67
<211>  22
<212>  DNA
<213>  Artificial Sequence

<220>
<223>  primer

<400>  67
aaccgcttca ctacttccaa ga                                               22


<210>  68
<211>  20
<212>  DNA

<213>  Artificial Sequence

<220>
<223>  primer

<400>  68
cgaaataagt cgggtagtgc                                                   20


<210>  69
<211>  21
<212>  DNA
<213>  Artificial Sequence

<220>
<223>  primer

<400>  69
cacttgcggt taattcgttg t                                                 21


<210>  70
<211>  21
<212>  DNA
<213>  Artificial Sequence

<220>
<223>  primer

<400>  70
ctttgatgga tgccctttgt a                                                 21


<210>  71
<211>  22
<212>  DNA
<213>  Artificial Sequence

<220>
<223>  primer

<400>  71
tagtcgtcat gggaaagaaa gg                                                22


<210>  72
<211>  23
<212>  DNA
<213>  Artificial Sequence

<220>
<223>  primer

<400>  72
cctatatata cgggtccgtg cag                                               23


<210>  73
<211>  21
<212>  DNA
<213>  Artificial Sequence

<220>

<223>  primer

<400>  73
taatcgccga aattgtacga c                                                21


<210>  74
<211>  21
<212>  DNA
<213>  Artificial Sequence

<220>
<223>  primer

<400>  74
gacatcacaa gggttggttt g                                                21


<210>  75
<211>  21
<212>  DNA
<213>  Artificial Sequence

<220>
<223>  primer

<400>  75
ccacttcgac tttcagtctc g                                                21


<210>  76
<211>  21
<212>  DNA
<213>  Artificial Sequence

<220>
<223>  primer

<400>  76
tgggttggac gacatacatc t                                                21


<210>  77
<211>  21
<212>  DNA
<213>  Artificial Sequence

<220>
<223>  primer

<400>  77
taatggttgg agcagcaaac t                                                21


<210>  78
<211>  22
<212>  DNA
<213>  Artificial Sequence

<220>
<223>  primer

<400>  78

cacgataaat tcagcactgg tc                                              22

<210>  79
<211>  21
<212>  DNA
<213>  Artificial Sequence

<220>
<223>  primer

<400>  79
aggttcccca ctcttctttg a                                               21

<210>  80
<211>  22
<212>  DNA
<213>  Artificial Sequence

<220>
<223>  primer

<400>  80
gaagcaaatg gaaaaggaac ac                                              22

<210>  81
<211>  22
<212>  DNA
<213>  Artificial Sequence

<220>
<223>  primer

<400>  81
agtgcagacg aagtaacacg aa                                              22

<210>  82
<211>  22
<212>  DNA
<213>  Artificial Sequence

<220>
<223>  primer

<400>  82
tcacgctctg taggatttga ac                                              22

<210>  83
<211>  22
<212>  DNA
<213>  Artificial Sequence

<220>
<223>  primer

<400>  83
ctctctcccg atgactgact ct                                              22

<210>  84
<211>  22
<212>  DNA
<213>  Artificial Sequence

<220>
<223>  primer

<400>  84
caggttcagc acgaaatcat aa                                                22


<210>  85
<211>  21
<212>  DNA
<213>  Artificial Sequence

<220>
<223>  primer

<400>  85
gaattgttct caagcgaatg g                                                 21


<210>  86
<211>  21
<212>  DNA
<213>  Artificial Sequence

<220>
<223>  primer

<400>  86
tttcgtgagt atgcggttct t                                                 21


<210>  87
<211>  21
<212>  DNA
<213>  Artificial Sequence

<220>
<223>  primer

<400>  87
aggcatggaa ggaatacgac t                                                 21


<210>  88
<211>  21
<212>  DNA
<213>  Artificial Sequence

<220>
<223>  primer

<400>  88
gccttgctct aaaggagctt g                                                 21


<210>  89
<211>  21
<212>  DNA

<213>  Artificial Sequence

<220>
<223>  primer

<400>  89
aagtggtgag gtttgcagag a                                                21


<210>  90
<211>  22
<212>  DNA
<213>  Artificial Sequence

<220>
<223>  primer

<400>  90
ggccagagct acttgggttt ag                                               22

## Description

### [CROSS-REFERENCE TO RELATED APPLICATIONS]

This application claims priority from prior Japanese Patent Application No. 2019-78905, filed on April 17, 2019, the entire contents of which are incorporated herein by reference.

### [BACKGROUND OF THE INVENTION]

### Technical Field

The present invention relates to a cytoplasmic male sterile plant of the genus Lactuca possessing improved low temperature growth ability.

### Background Art

Plant varieties include true breeding varieties and first filial hybrid (hereinafter referred to as "F1") varieties, and the F1 varieties are widely used for producing major crops. F1 varieties, which possess vigorous growth due to heterosis, have many advantages over true breeding varieties, such as improved growth rate and high yield. Further, the vigorous growing F1 varieties often have improvements in resistance to pests and adaptability to environment, such as cold and heat tolerance.

Further, the F1 varieties exhibit highly uniform phenotypes due to having heterozygous but identical genotypes. This increases marketability of their products. Further, favorable traits governed by dominant genes can be accumulated in parents of the F1 varieties, allowing rapid variety development.

Because of the presence of the advantages above, the F1 varieties have become the predominant cultivated varieties in the major crops.

When seeds of F1 varieties are produced, inbred lines are generally used as their parent lines. A seed parent and a pollen parent are selected based on their combination causing improved effects on hybrid vigour.

The seed parent requires emasculation to prevent self-pollination. However, manual emasculation needs an extremely large amount of labor. In this regard, using a cytoplasmic male sterility (hereinafter referred to as "CMS") line causing genetic male sterility as a seed parent, eliminates the labor for manual emasculation and enables economical mass production of F1 seeds. For the production of F1 seeds utilizing CMS, commercial production systems has been established for sunflower, sugar beet, potato, wheat, carrot, onion, bunching onion, cabbage, broccoli, daikon radish, Chinese cabbage, and others.

As for a plant of the genus Lactuca, there was no plant having CMS in a closely related species. Given this circumstance, the world's first CMS plant of the genus Lactuca was developed using a CMS of sunflower, which is a quite distantly related species, introducing CMS into a plant of the genus Lactuca by means of an asymmetric protoplast fusion technique (Patent Literature 1). Note that the conventional CMS plant of the genus Lactuca has been internationally deposited. The conventional CMS plant of the genus Lactuca, having very stable male sterility, eliminates the necessity of manual emasculation and enables the efficient production of F1 seeds.

In F1 breeding utilizing CMS, it is important that the cytoplasm exhibiting male sterility has minimal effects on traits other than male sterility.

For example, a T-type male sterile cytoplasm was introduced in maize and many F1 hybrid varieties were bred. However, in 1970, the T race of Cochliobolus heterostrophus appeared and, due to having susceptibility to this specific race, caused serious damage to the varieties with the T-type male sterile cytoplasm. This immediately ended the use of the T-type male sterile cytoplasm, leaving no choice but to go back to the conventional emasculation method, detasseling (Non Patent Literature 1).

Further, CMS of petunia has been known for a long time, and a causal gene thereof called S-pcf is widely used as a research material. However, varieties utilizing this CMS exhibit a delay in flowering and arrested development of the flower bud development, thus it is rarely used at present (Non Patent Literature 1).

As for CMS of daikon radish, Ogura CMS is the most widely used CMS. However, the presence of a fertility restoration gene within the species made it difficult to introduce CMS into some breeding lines. Subsequently, in daikon radish, NWB-CMS, having a different CMS factor, has been newly developed to improve the introduction rate of CMS (Patent Literature 2). Further, in daikon radish a new type of CMS has been developed in which an efficiency in attracting pollinating insects is improved by producing a small amount of sterile pollen (Patent Literature 3). These new types of daikon radish CMS have been developed by recurrent backcrossing using wild daikon radish as germplasm.

As shown by these examples, even CMS systems that have been known for a long time may be associated with unfavorable traits or be affected by male fertility restoration, making it difficult to use or limiting the use of CMS.

However, owing to a very high utility value for CMS, in a case where it is difficult to use existing CMS systems, new CMS sources have been looked for in order to develop CMS lacking defects like those described in the above examples.

The post-germination growth ability of the conventional CMS plant of the genus Lactuca (line name "50125-3-V1"), described in the above Patent Literature 1, under the optimal temperature condition (20°C) was comparable to that of a plant of the genus Lactuca with a normal cytoplasm. However, although not known at the time of describing Patent Literature 1, research conducted later found that the conventional CMS plant of the genus Lactuca might have reduced low temperature growth ability. Superiority or inferiority in the post-germination growth ability has a great impact on the quality of the subsequent seedlings, providing a very important agricultural trait. Thus, development of CMS lettuce improved in low temperature growth ability can further widen application of the CMS lettuce and significantly increase industrial usability of F1 lettuce.

The conventional CMS plant of the genus Lactuca described in Patent Literature 1 has excellent traits except that its low temperature growth ability is decreased. Thus, improving the unfavorable trait of low temperature growth ability using this CMS plant of the genus Lactuca was considered a shortcut to the development of improved CMS lettuce.

However, there has been no report on an effective method for improving an unfavorable trait from CMS in any plants. Thus, developing a CMS Lactuca improved in low temperature growth ability was considered very difficult.

### [Citation List]

### [Patent Literature]

PTL 1: WO 2007/049730 A
PTL 2: JP 3964368 B2
PLT 3: JP 5089764 B2

### [Non Patent Literature]

NPL 1: "Cytoplasmic male sterility and breeding technology", 1985, CMC Publishing Co., Ltd.
NPL 2: C. RAMBAUD, A. BELLAMY, A. DUBREUCQ, J.-C. BOURQUIN, and J. VASSEUR (1997) Plant breeding Volume 116, Issue 5 p481-486 "Molecular analysis of the fourth progeny of plants derived from cytoplasmic male sterile chicory cybrid"

### [SUMMARY OF THE INVENTION]

### [Technical Problem]

The present invention was made in view of the above-described problem in which the conventional CMS plant of the genus Lactuca has reduced low temperature growth ability, and an object of the present invention is to provide 1.) a CMS plant of the genus Lactuca having low-temperature growth ability and 2.) a method for producing F1 seed of a plant of the genus Lactuca having the low temperature growth ability using said CMS plant.

### [Solution to Problem]

As a result of intensive studies, the present inventors have found that it is possible to improve a cytoplasm, in particular, the mitochondrial genome, by performing asymmetric protoplast fusion (hereinafter also referred to as "asymmetric back fusion") using the conventional CMS plant of the genus Lactuca as a cytoplasm donor parent and a plant of the genus Lactuca with a normal cytoplasm as a cytoplasm acceptor parent. As a result, it is possible to develop a CMS plant of the genus Lactuca having low temperature growth ability and efficiently obtain F1 seed of the plant of the genus Lactuca having low temperature growth ability using said CMS plant.

The present invention is based on these findings.

Specifically, the present invention provides the following invention.
<1> A cytoplasmic male sterile plant of the genus Lactuca having low temperature growth ability comparable to that of a plant of the genus Lactuca with a normal cytoplasm, or a progeny thereof.
<2> A cytoplasmic male sterile plant of the genus Lactuca or the progeny thereof according to <1>, including DNA derived from the mitochondrial genome of a plant of the genus Helianthus in the mitochondrial genome.
<3> A cytoplasmic male sterile plant of the genus Lactuca or the progeny thereof according to <1> or <2>, being obtained by performing asymmetric protoplast fusion multiple times using the plant of the genus Lactuca with the normal cytoplasm as a cytoplasm acceptor parent.
<4> A cytoplasmic male sterile plant of the genus Lactuca or the progeny thereof according to any one of <1> to <3>, being obtained by performing the asymmetric protoplast fusion using an existing-cytoplasmic-male-sterile-plant of the genus Lactuca as a cytoplasm donor parent and the plant of the genus Lactuca with the normal cytoplasm as the cytoplasm acceptor parent.
<5> A cytoplasmic male sterile plant of the genus Lactuca including DNA derived from the mitochondrial genome of a plant of the genus Helianthus in the mitochondrial genome, or a progeny thereof, in which the cytoplasmic male sterile plant of the genus Lactuca or the progeny thereof is obtained by:
   performing asymmetric protoplast fusion multiple times using the plant of the genus Lactuca with a normal cytoplasm as a cytoplasm acceptor parent; or
   performing the asymmetric protoplast fusion using the existing-cytoplasmic-male-sterile-plant of the genus Lactuca as a cytoplasm donor parent and the plant of the genus Lactuca with the normal cytoplasm as the cytoplasm acceptor parent.
<6> A cytoplasmic male sterile plant of the genus Lactuca or the progeny thereof according to any one of <1> to <5>, in which the cytoplasmic male sterile plant of the genus Lactuca is derived from Lactuca sativa L. or an interspecific hybrid plant of the plant of the genus Lactuca.
<7> A cytoplasmic male sterile plant of the genus Lactuca including mitochondrial genome of a plant which is specified by a deposition number FERM BP- 22373, or a progeny thereof.
<8> A cytoplasmic male sterile plant of the genus Lactuca which is specified by a deposition number FERM BP- 22373, or a progeny thereof.
<9> A part of a plant body of the cytoplasmic male sterile plant of the genus Lactuca or the progeny thereof according to any one of <1> to <8>.
<10> A seed of the cytoplasmic male sterile plant of the genus Lactuca or the progeny thereof according to any one of <1> to <8>.
<11> Mitochondrial genome included in the cytoplasmic male sterile plant of the genus Lactuca or the progeny thereof according to any one of <1> to <8>, the part of the plant body according to <9>, or the seed according to <10>.
<12> An asymmetric back-fusion method of a plant, comprising performing asymmetric protoplast fusion once or multiple times using: a cytoplasmic male sterile plant of the genus Lactuca as a cytoplasm donor parent, the cytoplasmic male sterile plant of the genus Lactuca being a plant obtained by the asymmetric protoplast fusion or a progeny thereof; and
   a plant of the genus Lactuca with a normal cytoplasm as a cytoplasm acceptor parent, the plant of the genus Lactuca with the normal cytoplasm being one of the plants used in the initial asymmetric protoplast fusion.
<13> A method for improving the mitochondrial genome in a cytoplasm of a plant of the genus Lactuca using the method according to <12>.
<14> A method for producing a cytoplasmic male sterile plant of the genus Lactuca having low temperature growth ability comparable to that of a plant of the genus Lactuca with a normal cytoplasm, or a progeny thereof, the method comprising performing asymmetric protoplast fusion using the existing-cytoplasmic-male-sterile-plant of the genus Lactuca as a cytoplasm donor parent and the plant of the genus Lactuca with the normal cytoplasm as a cytoplasm acceptor parent.
<15> The method according to <14>, in which the cytoplasmic male sterile plant of the genus Lactuca is Lactuca sativa L. or a plant derived from an interspecific hybrid plant of the plant of the genus Lactuca.
<16> A method for producing a first filial hybrid seed, comprising crossing the cytoplasmic male sterile plant of the genus Lactuca or the progeny thereof according to any one of <1> to <8> as a seed parent with a plant of the genus Lactuca crossable with the former plant as a pollen parent and producing the first filial hybrid seed from the seed parent after the crossing.
<17> The method according to <16>, in which the pollen parent is Lactuca sativa L. or an interspecific hybrid plant of the plant of the genus Lactuca.
<18> A first filial hybrid seed developed by the method according to <16> or <17>, or a first filial hybrid plant grown from the seed, or a progeny thereof, or a part of a plant body thereof.
<19> A method for producing a plant of the genus Lactuca having a favorable trait and exhibiting cytoplasmic male sterility, the method comprising performing recurrent backcross between the cytoplasmic male sterile plant of the genus Lactuca or the progeny thereof according to any one of <1> to <8> and a plant of the genus Lactuca having the favorable trait for cytoplasmic replacement.
<20> The method according to <19>, in which the plant of the genus Lactuca having the favorable trait is derived from Lactuca sativa L.

### [Effects of the Invention]

According to the present invention, it is possible to produce an improved CMS plant of the genus Lactuca, improved in its low temperature growth ability. Using the improved CMS plant of the genus Lactuca of the present invention makes it possible to efficiently produce F1 seed of plants of the genus Lactuca having low temperature growth ability.

Further, according to the asymmetric back-fusion method of the present invention, it is possible to improve the cytoplasm of a plant, in particular, the mitochondrial genome.

### [BRIEF Description OF DRAWINGS]

Fig. 1 shows a germination test result on Day 17 after sowing "Tell me" with a normal cytoplasm (A) and "Tell me" with the conventional CMS cytoplasm (B) under a greenhouse environment in which a nighttime temperature is set to 0°C and a daytime temperature is set to 20°C.
Fig. 2 shows a germination test result on Day 17 after sowing "V lettuce" with a normal cytoplasm (A) and "V lettuce" with the conventional CMS cytoplasm (B) under the greenhouse environment in which the nighttime temperature is set to 0°C and the daytime temperature is set to 20°C.
Fig. 3 shows a germination test result on Day 16 after sowing "V lettuce" with a normal cytoplasm (A) and "V lettuce" with the conventional CMS cytoplasm (B) in an artificial climate chamber set to an optimum growth temperature condition (the nighttime temperature of 20°C, the daytime temperature of 20°C, 12-hour illumination).
Fig. 4 shows a germination test result on Day 17 after sowing "V lettuce" with a normal cytoplasm (A) and "V lettuce" with the conventional CMS cytoplasm (B) in an artificial climate chamber set to a low temperature condition (the nighttime temperature of 5°C, the daytime temperature of 20°C, 12-hour light period).
Fig. 5 shows a germination test result on Day 17 after sowing "V lettuce" with a normal cytoplasm (A) and "V lettuce" with an improved CMS cytoplasm (B) under the greenhouse environment in which the nighttime temperature is set to 0°C and the daytime temperature is set to 20°C.
Fig. 6 shows a germination test result on Day 17 after sowing "M8-039" with a normal cytoplasm (A) and "M8-039" with an improved CMS cytoplasm (B) under the greenhouse environment in which the nighttime temperature is set to 0°C and the daytime temperature is set to 20°C.
Fig. 7 shows a germination test result on Day 17 after sowing "Steady" with a normal cytoplasm (A) and "Steady" with an improved CMS cytoplasm (B) under the greenhouse environment in which the nighttime temperature is set to 0°C and the daytime temperature is set to 20°C.
Fig. 8 shows a germination test result on Day 17 after sowing "Steady" with a normal cytoplasm (A) and "Steady" with an improved CMS cytoplasm (B) under a low temperature environment in the artificial climate chamber (the nighttime temperature of 5°C, the daytime temperature of 16°C, 12-hour light period).
Fig. 9 shows a state in which "Steady" with a normal cytoplasm (A) and "BF2MS1S" (B) in BC7 generation are planted in a field (Azumino, Nagano, Japan) and grown until the head-forming period.

### [DETALED DESCRIPTION OF THE INVENTION]

Hereinafter, the present invention will be described in detail.

Cytoplasmic Male Sterile Plant of The Genus Lactuca Improved in Low Temperature Growth Ability and Progeny Thereof

The present invention relates to a cytoplasmic male sterile plant of the genus Lactuca improved in low temperature growth ability compared with that in the prior art, or a progeny thereof. This invention can be expressed, as described above, as a cytoplasmic male sterile plant of the genus Lactuca having low temperature growth ability comparable to that of a plant of the genus Lactuca with a normal cytoplasm, or a progeny thereof.

In the present specification, the term "low temperature growth ability" means growth ability of a seedling under a low temperature condition. The low temperature described herein refers to a temperature condition lower than the optimal temperature condition under which a plant of the genus Lactuca is originally grown, and includes a condition in which the average daily temperature is low and a condition in which a temperature is low only in a part of time period during a day (e.g., at nighttime). Specific examples of the low temperature include a condition in which a daytime temperature is 20°C and a nighttime temperature is lower than the daytime temperature, for example, from 5°C to 0°C.

The low temperature growth ability can be quantitatively evaluated by cultivating plants to be compared under the same low temperature condition, measuring and comparing the weight of an above-ground part of each seedling, and then comparing these results with those obtained by cultivating the plants under the appropriate temperature condition.

In the present invention, the term "normal cytoplasm" is typically used to mean that the cytoplasm is normal with no effect on male fertility as compared with the cytoplasm of the plant exhibiting male sterility, that is, the male sterile cytoplasm.

Further, the term "comparable" in the phrase "low temperature growth ability comparable to that of the plant of the genus Lactuca with a normal cytoplasm" means a case in which, when the low temperature growth ability is measured on the basis of the weight of the above-ground part of the seedling, a measured value of the plant to be compared varies within a range of 25% or less (preferably 20% or less, more preferably 15% or less, further preferably 10% or less) of a value of the plant of the genus Lactuca with a normal cytoplasm. Thus, for example, in comparison with a value of the weight of the above-ground part of the seedling of the "plant of the genus Lactuca with a normal cytoplasm", when the measured value of the plant to be compared is 90% of the value of the normal plant, this example corresponds to the case where the aforementioned variance is 10%. Being comparable does not exclude a case of exceeding the low temperature growth ability of the "plant of the genus Lactuca with a normal cytoplasm".

In the present specification, the term "progeny" includes not only a progeny of the cytoplasmic male sterile plant of the genus Lactuca obtained by using a maintainer line, but also a crossbreed obtained by crossing the cytoplasmic male sterile plant of the genus Lactuca of the present invention with a plant of the genus Lactuca crossable with the aforementioned plant. Thus, the "progeny" includes, for example, a plant obtained by crossing the cytoplasmic male sterile plant of the genus Lactuca of the present invention as a seed parent (female parent) with a plant of the genus Lactuca crossable with the aforementioned plant as a pollen parent (male parent). Further, the "progeny" includes, for example, a plant obtained by protoplast fusion between the cytoplasmic male sterile plant of the genus Lactuca of the present invention and a plant capable of being fused to the aforementioned plant of the genus Lactuca, and a plant obtained by interspecific and intergeneric hybridization.

The "plant of the genus Lactuca" described herein is preferably Lactuca sativa L., L. serriola, L. aculeate, L. scarioloides, L. azerbaijanica, L. georgica, L. dregeana, L. altaica, L. saligna, L. virosa, L. tatarica, L. indica, or L. debilis, or a plant obtained by interspecific hybridization therebetween. Of these, Lactuca sativa L., which is a cultivated species of the genus Lactuca, or a plant obtained by interspecific hybridization between plants of the genus Lactuca is preferable, and Lactuca sativa L. is more preferable.

According to a preferred embodiment of the present invention, the cytoplasmic male sterile plant of the genus Lactuca of the present invention or a progeny thereof has DNA derived from the mitochondrial genome of a plant of the genus Helianthus in the mitochondrial genome.

The "plant of the genus Helianthus" described herein is preferably, among the plants of the genus Helianthus, Helianthus annuus L., which is a cultivated variety of sunflower, or a cytoplasmic replacement line of Helianthus annuus L. having a cytoplasm derived from H. petiolaris, H. argophyllus, H. debilis, H. decapetalus, H. giganteus, H. rigidus, H. salicifolius, H. anomalus, H. bolanderi, H. exilis, H. maximiliani, H. neglectus, H. praecox or H. tuberosus.

In the present specification, the asymmetric protoplast fusion refers to a type of protoplast fusion in which the nuclear genome of one of the protoplasts isolated for the protoplast fusion is inactivated in advance, and then used for the protoplast fusion. In this asymmetric protoplast fusion, a protoplast which the nuclear genome is inactivated to supply its cytoplasm to a fusion cell in the protoplast fusion, is referred to as a cytoplasm donor parent. Further, a protoplast which the nuclear genome is maintained without being inactivated to receive the cytoplasm from the cytoplasm donor parent is referred to as a cytoplasm acceptor parent.

Further, the asymmetric back fusion (or asymmetric back-protoplast fusion) described herein refers to a type of protoplast fusion in which a plant obtained by the asymmetric protoplast fusion or a progeny thereof is used as the cytoplasm donor parent and one of the plants used in the initial asymmetric protoplast fusion is used as the cytoplasm acceptor parent to further perform the asymmetric protoplast fusion one or more times (preferably one time). That is, in the asymmetric back fusion, the asymmetric protoplast fusion is performed two or more times, including the initial fusion.

When the cytoplasmic male sterile plant of the genus Lactuca of the present is obtained, the plant of the genus Lactuca with a normal cytoplasm is desirably used as the cytoplasm acceptor parent in the asymmetric protoplast fusion. Thus, according to a preferred embodiment of the present invention, the cytoplasmic male sterile plant of the genus Lactuca of the present invention can be obtained by performing the asymmetric protoplast fusion using the plant of the genus Lactuca with a normal cytoplasm as the cytoplasm acceptor parent multiple times, that is, two or more times. Alternatively, according to another preferred embodiment of the present invention, the cytoplasmic male sterile plant of the genus Lactuca of the present invention can be obtained by performing the asymmetric protoplast fusion using an existing-cytoplasmic-male-sterile-plant of the genus Lactuca as the cytoplasm donor parent and the plant of the genus Lactuca with a normal cytoplasm as the cytoplasm acceptor parent. In this disclosure, an "existing-cytoplasmic-male-sterile-plant of the genus Lactuca" means a cytoplasmic male sterile plant of the genus Lactuca before being improved by the present invention, that is, a conventional cytoplasmic male sterile plant of the genus Lactuca. In the present invention, the existing-cytoplasmic-male-sterile-plant of the genus Lactuca preferably refers to the one which has room for improvement in low temperature growth ability, that is, the one having the reduced low temperature growth ability compared with the plant of the genus Lactuca with a normal cytoplasm.

Therefore, according to another aspect of the present invention, the cytoplasmic male sterile plant of a genus Lactuca including DNA derived from mitochondrial genome of a plant of a genus Helianthus in the mitochondrial genome, or a progeny thereof, according to the present invention, is obtained by:
performing asymmetric protoplast fusion multiple times using the plant of the genus Lactuca with a normal cytoplasm as a cytoplasm acceptor parent; or
performing the asymmetric protoplast fusion using the existing-cytoplasmic-male-sterile-plant of the genus Lactuca as a cytoplasm donor parent and the plant of the genus Lactuca with the normal cytoplasm as the cytoplasm acceptor parent.

According to a more preferred embodiment of the present invention, the cytoplasmic male sterile plant of the genus Lactuca of the present invention or the progeny thereof is a plant of the genus Lactuca having the mitochondrial genome of a plant specified by the deposition number FERM BP-22373 (described below) or a progeny thereof, more preferably a plant of the genus Lactuca specified by the deposition number FERM BP-22373 or a progeny thereof.

According to another embodiment of the present invention, an asymmetric back-fusion method of developing a plant is provided. This asymmetric back-fusion method includes performing, once or multiple times, the asymmetric protoplast fusion using a plant obtained by the asymmetric protoplast fusion or a progeny thereof and one of the plants used in the aforementioned initial asymmetric protoplast fusion. Here, preferably, an asymmetric back-fusion method of a plant uses a cytoplasmic male sterile plant of the genus Lactuca as a cytoplasm donor parent, the cytoplasmic male sterile plant of the genus Lactuca being a plant obtained by the asymmetric protoplast fusion or a progeny thereof, and a plant of the genus Lactuca with a normal cytoplasm as a cytoplasm acceptor parent, the plant of the genus Lactuca with the normal cytoplasm being one of the plants used in the initial asymmetric protoplast fusion.

According to yet another embodiment of the present invention, a method for improving the mitochondrial genome in the cytoplasm of a plant using the aforementioned asymmetric back-fusion method is provided.

In the present specification, a "part of a plant body" of the cytoplasmic hybrid plant of the genus Lactuca or a progeny thereof includes one or more cells or a cytoplasm of one or more cells of the aforementioned plant body, specifically, an organ or tissue such as a flower, a leaf, a stem, a root, or the like, a cell (including a protoplast prepared from the cell) or a cytoplasm derived from these organs and tissues, or a population of the aforementioned cells or cytoplasms.

Method for Developing Cytoplasmic Male Sterile Plant of Genus Lactuca Improved in Low Temperature Growth Ability

An improved cytoplasmic male sterile plant of the genus Lactuca of the present invention can be developed, for example, by the following procedures.
(1) Preparation of protoplast
   (i) Isolation of protoplast of plant with normal cytoplasm
   (ii) Isolation of protoplast of cytoplasmic male sterile plant
(2) Fusion treatment of protoplasts
(3) Culture of fused hybrid cell
(4) Regeneration of plant body from callus
(5) Selection of cytoplasmic hybrid plant
(6) Selection of superior line
(7) Recurrent backcross of superior line
(8) Use of cytoplasmic male sterile plant and production of F1 seed

Note that, in the present specification, the term "production method" can be referred to as "development method". That is, the terms "development" and "production" described herein are used with the same meaning.

More specifically, each of these steps is as follows.

### (1) Preparation of protoplast

### (i) Isolation of protoplast of plant of the genus Lactuca with normal cytoplasm

In the present invention, the "plant of the genus Lactuca" used as a cytoplasm acceptor parent (recipient) is preferably Lactuca sativa L., L. serriola, L. aculeate, L. scarioloides, L. azerbaijanica, L. georgica, L. dregeana, L. altaica, L. saligna, L. virosa, L. tatarica, L. indica, or L. debilis, or a plant obtained by interspecific hybridization therebetween. Of these, Lactuca sativa L., which is the cultivated species of the genus Lactuca, is more preferable.

A cellular tissue used to obtain a protoplast is desirably a mesophyll tissue, which provides high yield and is highly active in cell division. However, other tissues such as a hypocotyledonous stem, a stem, and a callus may be used as a material.

A method for isolating the protoplast may be one of commonly used methods known in the art (e.g., methods described in Matsumoto, E, Plant cell reports, 1991. vol.9(10) and the like) without particular limitations. The following procedures are mentioned as a specific example. However, the present invention is not necessarily limited to these procedures.

First, a cellular tissue of a plant of the genus Lactuca is cut into fine pieces and subjected to an enzyme treatment using an enzyme solution for isolating protoplast to isolate a protoplast.

This solution is an inorganic salt buffer that mainly contains a cell wall degrading enzyme and an osmotic pressure regulator. The cell wall degrading enzyme is not particularly limited as long as it can be used for degrading a plant cell wall. However, examples thereof include cellulase, hemicellulase, and pectinase. In the present invention, a combination of Cellulase Y-C and Macerozyme R-10 is preferably used. As the osmotic pressure regulator, a common sugar alcohol, for example, mannitol, sorbitol, glucose, or the like, can be used. Of these, mannitol is preferable and mannitol with a concentration of from 0.3 M to 0.7 M is particularly preferable. Further, an inorganic salt is desirably added to the enzyme solution to stabilize the membrane of the protoplast. For example, CPW salts (Cocking and Peberdy, 1974) which composition is described in Table 1 below are preferably added. The enzyme treatment is preferably performed at from 25°C to 30°C for 8 to 20 hours under a steady condition.

**Table 1 Composition of CPW salt solution**

| | |
|---|---|
| KH₂PO₄ | 27.2mg/l |
| KNO₃ | 101.0 mg/l |
| CaCl₂ • 2H₂O | 1,480.0 mg/l |
| MgSO₄ | 246.0 mg/l |
| KI | 0.16 mg/l |
| CuSO₄ • 5H₂O | 0.025. mg/l |
| Mannitol | 0.6 M |
| pH | 5.8 |

The protoplasts isolated by the enzyme treatment are filtered through nylon mesh with a pore diameter of from 30 to 100 µm and subjected to centrifugal separation to collect the protoplasts by removing the enzyme solution. Next, the protoplasts are suspended in a washing solution to wash the protoplasts. For the washing solution, a solution prepared by adding a sugar alcohol as an osmotic pressure regulator to the commonly used CPW salt solution can be used.

Next, an inactivation treatment is performed to prevent division of the protoplasts of the plant of the genus Lactuca alone. The inactivation treatment can be performed by suspending the protoplasts in the CPW salt solution in which an iodine compound such as iodoacetic acid or iodoacetamide is dissolved, or the like. In the present invention, it is preferable that the protoplasts are suspended in the CPW solution in which the concentration of iodoacetamide is adjusted to from 5 mM to 30 mM and the treatment is performed for 5 to 20 minutes.

Next, the washing operation with the CPW salt solution are preferably repeated one to three times using centrifugal separation. The protoplast suspension is contaminated with pieces of vessels and cells, thus the protoplasts are preferably further purified by density gradient centrifugation or the like. As for a reagent that can be used for purification, a sugar, a synthetic colloid, or the like can be mentioned. However, in the present invention, a sucrose solution is preferably used, and a 15% to 20% sucrose solution is particularly preferable. After purification of the protoplasts, the cell density is measured with a hemocytometer, and the liquid volume is adjusted with the CPW salt solution to achieve the cell density suitable for the protoplast fusion. The cell density of the protoplasts is preferably from 1 x 10⁵ to 1 × 10⁷ cells/ml, and the CPW salt solution is preferably used for adjusting the liquid volume.

### (ii) Isolation of protoplast of conventional CMS plant of the genus Lactuca

The CMS plant of the genus Lactuca used as the cytoplasm donor parent (donor) is not particularly limited as long as it has cytoplasmic male sterility. However, the plant of the genus Lactuca preferably has stable CMS. Specifically, for example, the conventional CMS plant of the genus Lactuca described in Patent Literature 1 (deposition number FERM BP-10647) is preferably used as the cytoplasm donor parent.

Isolation of the protoplasts of the conventional CMS plant of the genus Lactuca can be performed by a method similar to, for example, that of the isolation of the protoplasts of the plant of the genus Lactuca described above.

The protoplasts of the conventional CMS plant of the genus Lactuca thus isolated are preferably subjected to a radiation treatment to inactivate the nuclear genes before use.

Examples of a radiation used in the radiation treatment include an X-ray, a gamma ray, and an ultraviolet ray. However, it is not particularly limited as long as the nuclear genes can be inactivated. The radiation dose is preferably as low as possible in a range capable of inactivating the nuclear genes. For example, the radiation dose is preferably from 100 Gy to 900 Gy when a soft X-ray is radiated in the present invention.

### (2) Fusion treatment of protoplasts

Next, two kinds of the protoplasts obtained in the above are mixed for performing protoplast fusion.

As a fusion method, commonly used methods including, for example, a known electrofusion method (Planta, 151, 26-32, 1981), PEG (polyethylene glycol) method (Planta, 120, 215-227, 1974), and dextran method (Jap. J. Genet., 50, 235, 1975) can be mentioned, without being particularly limited thereto. In the present invention, the PEG method is preferably used.

### (3) Culture of fused hybrid cell

A cell obtained by the fusion treatment is preferably cultured in a medium suitable for culturing a protoplast derived from a plant of the genus Lactuca.

Various methods are known as a method for culturing the protoplast derived from the plant of the genus Lactuca. In the present invention, a method obtained by modifying the method in Patent Literature 1 is preferably used without being limited thereto.

In Example 1 in Patent Literature 1, the asymmetric protoplast fusion is performed using a combination of the protoplast of the genus Helianthus and the protoplast of the plant of the genus Lactuca. On the other hand, in the present invention, the asymmetric protoplast fusion is performed using a combination of the protoplasts both from the plants of the genus Lactuca, making it possible to culture the resulting fusion cell more suitably by examining the optimal type and concentration of plant growth regulators.

As a specific example of such an optimal condition, the fusion cell can be efficiently cultured by appropriately combining the plant growth regulators, α-naphthaleneacetic acid (NAA), N-(2-chloro-4-pyridyl)-N'-phenylurea (4-CPPU), and thidiazuron (TDZ).

### (4) Regeneration of plant body from callus

The fusion cell is cultured and allowed to undergo cell division. When a callus is visually confirmed, the callus is transferred to a regeneration medium to induce regeneration.

As a regeneration medium, a commonly used medium can be used. Although there is a difference in responses depending on the plants of the genus Lactuca as a material and conditions of the callus, for example, an MS medium containing from 0.3 to 1.0 mg/l 4-CPPU, or the like, is preferably used. A regenerated shoot is transferred to a rooting medium, for example, a half-concentration MS medium, and allowed to generate a root, thereby regenerating a plant body. The regenerated plant body is grown in a greenhouse after acclimatization.

### (5) Selection of cytoplasmic hybrid plant

DNA is extracted from the plant body regenerated by the above procedures and leaves of the conventional CMS plant of the genus Lactuca and the plant of the genus Lactuca with a normal cytoplasm used as a material. For the purpose of efficiently selecting a cytoplasmic hybrid plant, detection by a PCR method is preferably performed using a marker capable of specifically amplifying a mitochondrial DNA sequence that can distinguish between the conventional CMS plant of the genus Lactuca and the plant of the genus Lactuca with a normal cytoplasm.

The marker used in the PCR is not particularly limited as long as it can specifically amplify only a mitochondrial DNA sequence derived from the conventional CMS plant of the genus Lactuca. In the present invention, as described above, the conventional CMS plant of the genus Lactuca described in Patent Literature 1 (deposition number FERM BP-10647) is desirably used as the cytoplasm donor parent. In this case, detection in the PCR method can be performed using a marker capable of specifically amplifying the atp6 gene derived from sunflower.

The cytoplasmic hybrid plant can be efficiently selected by performing a PCR analysis using the above marker capable of specifically amplifying the mitochondrial DNA sequence.

Note that selection of the cytoplasmic hybrid plant can be performed by referring to the procedures in Patent Literature 1 accordingly as needed.

Conformation by the PCR method described above is desirably performed at the callus stage. However, it may be performed at other stages, for example, using the acclimated plant body. Further, it is also desirable that a ploidy test is performed to select for diploid while eliminating polyploidy by measuring a DNA content with flow cytometry, observing chromosomes, or the like. Recombination of the mitochondrial genome caused by the asymmetric protoplast fusion occurs very frequently and randomly, thus three hundred or more of the diploid cytoplasmic hybrid plants are desirably developed.

### (6) Selection of superior line

The cytoplasmic hybrid plant thus obtained is grown and allowed to bloom, and a line having a male sterile trait without exhibiting morphological abnormalities in other organs is selected. The selected male sterile individual is crossed with pollen of a plant of the genus Lactuca with a normal cytoplasm to produce a progeny seed.

Note that selection of the superior line can be performed by referring to the procedures in Patent Literature 1 accordingly as needed.

Next, to select a line superior in low temperature growth ability, sowing is performed under a low temperature condition. As a temperature condition, it is desirable to set a condition in which the nighttime temperature is from 0°C to 5°C and a daytime temperature is from 15°C to 20°C. The low temperature growth ability of the developed CMS line is compared with the plant of the genus Lactuca with a normal cytoplasm as a control, and an individual exhibiting the comparable or better low temperature growth ability is selected.

In a case where the individual exhibiting the comparable or better low temperature growth ability is not obtained, it is desirable that an individual exhibiting relatively better low temperature growth ability is selected and used as the cytoplasm donor parent to repeat the asymmetric protoplast fusion. In a case where the individual exhibiting the low temperature growth ability comparable to or better than that of the plant of the genus Lactuca with a normal cytoplasm is obtained, a transition is made to the next step of recurrent backcross.

### (7) Recurrent backcross of superior line

The cytoplasmic hybrid plant obtained by the protoplast fusion usually exists in a heteroplasmy state and thus tends to have variations in traits even in a backcrossed progeny. As a result, a progeny of the cytoplasmic hybrid may have different characteristics at the cell level, and selection of the superior CMS line is preferably performed on an individual-by-individual basis. Non Patent Literature 2 described above suggests that five generations or more of recurrent backcross is required for allowing the cytoplasm to reach a homoplasmy state.

Thus, in order to create the CMS line having low temperature growth ability, selection using the low temperature growth ability as an indicator and backcrossing are preferably repeated for five generations or more until the low temperature growth ability is stabilized.

When a CMS line having the stabilized low temperature growth ability is obtained, this line is selected as an improved CMS line (that is, a cytoplasmic male sterile plant of the genus Lactuca improved in low temperature growth ability) for use in actual breeding.

### (8) Use of cytoplasmic male sterile plant and production of F1 seed

For producing F1 seeds, inbred lines are generally used as both parental lines. Both parental lines, in which a seed parent and a pollen parent are defined, need to be artificially crossed to each other. This requires emasculation to prevent self-fertilization of the seed parent. In the plant of the genus Lactuca, it is difficult to perform artificial emasculation due to the structure of flower, thus CMS is preferably used.

The male sterility of the improved CMS line is passed on through cytoplasmic inheritance, making it possible to easily convert any superior plant of the genus Lactuca (that is, the plant of the genus Lactuca having a favorable trait) to the CMS plant by performing the recurrent backcross using the superior plant of the genus Lactuca as a pollen parent and the improved CMS line as a seed parent, thereby performing nuclear replacement. That is, using the improved CMS line deposited by the present invention makes it possible to convert any crossable plant of the genus Lactuca to the superior CMS plant without using the asymmetric back-fusion technique, which requires a high degree of skill.

In the recurrent backcross, any superior plant of the genus Lactuca converted to the CMS plant is used as a seed parent and crossed with any pollen parent by hand pollination or insect pollination, so that an F1 seed of the plant of the genus Lactuca having the low temperature growth ability can be efficiently obtained.

Performing F1 breeding of lettuce by the above procedures makes it possible to perform rapid breeding of a lettuce variety which is imparted with a superior trait, without losing the low temperature growth ability, and create a highly marketable lettuce variety with excellent product uniformity, environmental adaptability, and the like.

### [EXAMPLES]

The present invention will be described in detail by way of examples below. However, the present invention is not limited to these examples.

### Example 1: Development of improved CMS plant of the genus Lactuca

### (1) Preparation of protoplast

### (i) Isolation of protoplast of plant of the genus Lactuca with normal cytoplasm

As the plant of the genus Lactuca with a normal cytoplasm, a lettuce variety "Steady" (available from Tsuruta Seed) was used.

First, sterilized seeds were plated on an MS medium supplemented with 10 g/l sucrose and 8 g/l agar and cultured for one month at 20°C with 16-hour illumination. Approximately 1 g of opened true leaves were harvested and cut into fine pieces of about 2 mm in size. Then, they were soaked in 10 ml of a CPW salt solution containing 0.4% Cellulase Y-C, 0.4% Macerozyme R-10, and mannitol and left standing at 25°C for 16 hours.

An enzyme solution including the leaf tissues was filtered through 92 µm nylon mesh to remove cell debris. The protoplast suspension thus obtained was transferred to a centrifuge tube and subjected to centrifugal separation at 800 rpm for 5 minutes. The protoplasts obtained by removing the supernatant were suspended in 5 ml of the CPW salt solution containing 15 mM iodoacetamide and incubated at 25°C for 15 minutes. After incubation, the iodoacetamide-treated protoplast suspension was subjected to centrifugal separation at 800 rpm for 5 minutes to remove the supernatant. To the protoplast suspension, 10 ml of the CPW salt solution was added, and then the resulting mixture was subjected to centrifugal separation at 800 rpm for 5 minutes to remove the supernatant. This operation was repeated three times to wash the protoplasts.

The washed protoplast suspension was subjected to centrifugal separation at 800 rpm for 5 minutes to remove the supernatant, and the protoplasts were suspended by adding 2 ml of the CPW salt solution. In a new centrifuge tube, 5 ml of the CPW salt solution including 20% sucrose was put, and the protoplast suspension described above was overlaid on the CPW salt solution, followed by centrifugal separation at 800 rpm for 5 minutes. Cell debris settled to the bottom of the centrifuge tube. Purified protoplasts moved up to the upper layer corresponding to the CPW salt solution layer were collected into a new centrifuge tube using a Pasteur pipette. A small amount of the suspension was taken out for determining the cell density of the protoplasts using a hemacytometer. The CPW solution was added to the suspension to adjust the cell density to 1 x 10⁶ cells/ml.

### (ii) Isolation of protoplast of the conventional CMS lettuce

The conventional CMS lettuce, a line name "50125-3-V1" (deposition number FERM BP-10647) described in Patent Literature 1 was used.

Note that "50125-3-V1" described herein is a progeny obtained by crossing a CMS lettuce line "50125-3" to "V lettuce" (available from Kaneko Seeds Co., Ltd.). The seed of "50125-3-V1" has been internationally deposited at the International Patent Organism Depositary of the independent administrative corporation, National Institute of Advanced Industrial Science and Technology (Tsukuba Central 6, 1-1-1 Higashi, Tsukuba, Ibaraki, Japan) on July 31, 2006 (identification reference given by the depositors: SSC-LET-06-001, deposition number: FERM BP-10647) (Patent Literature 1).

First, sterilized seeds were plated on an MS medium supplemented with 10 g/l sucrose and 8 g/l agar and cultured for one month at 20°C with 16-hour illumination. Approximately 1 g of opened true leaves were harvested and cut into fine pieces of about 2 mm in size. Then, they were soaked in 10 ml of a CPW salt solution containing 0.4% Cellulase Y-C, 0.4% Macerozyme R-10, and mannitol and left standing at 25°C for 16 hours.

An enzyme solution including the leaf tissues was filtered through 92 µm nylon mesh to remove cell debris. The protoplasts were transferred to a plastic petri dish using a Pasteur pipette and irradiated with soft X-rays at a dose of 900 Gy.

The obtained protoplast suspension was transferred to a centrifuge tube and subjected to centrifugal separation at 800 rpm for 5 minutes to remove the supernatant, and the protoplasts were suspended by adding 2 ml of the CPW salt solution. Into a new centrifuge tube, 5 ml of the CPW salt solution including 20% sucrose was put, and the protoplast suspension described above was overlaid on the CPW salt solution, followed by centrifugal separation at 800 rpm for 5 minutes. Cell debris were settled to the bottom of the centrifuge tube. Purified protoplasts moved up to the upper layer corresponding to the CPW salt solution layer were collected into a new centrifuge tube using a Pasteur pipette. A small amount of the suspension was taken out for determining the cell density of the protoplasts using a hemacytometer. The CPW salt solution was added to the suspension to adjust the cell density to 1 x 10⁶ cells/ml.

### (2) Fusion treatment of protoplasts

The iodoacetamide-treated lettuce protoplast suspension and the conventional CMS lettuce protoplast suspension subjected to the soft X-ray irradiation were mixed at a ratio of 1:3, and 2 ml of the mixture was dropped onto the center of a 9 cm dish. After being left standing for 30 minutes, 3 ml of a PEG solution (500 g/l polyethylene glycol #6000 (nacalai tesque, Inc.), 1,500 mg/l CaCl₂-2H₂O, 100 mg/l KH₂PO₄, pH 5.5) was added dropwise around the protoplast mixture.

One minute later, 3.5 ml of the CPW salt solution was added dropwise around the protoplast mixture. Further 2 minutes later, 3.5 ml of the CPW salt solution was added dropwise around the protoplast mixture. Five minutes later, the liquid added dropwise was carefully removed by suction from the edge of the dish, and then 20 ml of the CPW salt solution was added at the edge of the dish. This operation of washing with the CPW salt solution was repeated three times at five-minute intervals.

### (3) Culture of fused hybrid cell

After removing the washing solution, 10 ml of a half-concentration MS medium (pH 5.8) containing 2.7 g/l disodium succinate hexahydrate, 0.5 g/l casamino acid, 0.1 mg/l NAA, 1.0 mg/l 4-CPPU, 0.1 mg/l TDZ, and 0.3 M sucrose with NH₄NO₃ reduced to 200 mg/l (hereinafter, referred to as a lettuce protoplast culture medium) was added to the protoplasts, and they were cultured at 25°C in dark.

Three days after the start of the culture, 5 ml of a four-times concentrated lettuce protoplast culture medium (sucrose with a concentration of 0.3 M) and 5 ml of a 0.3 M sucrose solution (pH 5.8) containing 0.6% gellan gum were mixed to prepare a gel medium in a semi-solid state, and the culture was continued in this medium.

Ten days after the start of the culture, 10 ml of the medium including the fused hybrid cells were transferred along with the gel into 10 ml of the lettuce protoplast culture medium in which the sucrose concentration was modified to 0.15 M. Twenty days after the start of the culture, a callus became visible to the naked eye. The callus thus cultured was transferred to the lettuce protoplast culture medium (solid medium, pH 5.8) containing 0.2 M sucrose and 0.3% gellan gum.

### (4) Regeneration of plant body from callus

Thirty days after the start of the culture, the callus grown to a size of about 2 mm was transferred to the MS medium (pH 5.8) containing 0.3 mg/l 4-CPPU, 1.0% sucrose, and 0.8% agar.

Forty to sixty days after the start of the culture, a shoot regenerated from the callus was transferred to the 1/2 MS medium (pH 5.8) containing 1.0% sucrose and 0.8% agar, thereby allowing the shoot to generate a root. Approximately three hundred or more of the cytoplasmic hybrid plants were regenerated.

The cytoplasmic hybrid plants were transferred to vermiculite, acclimated, and grown in a greenhouse.

### (5) Selection of cytoplasmic hybrid plants having mitochondrial gene specific to conventional CMS lettuce

In order to detect DNA specific to the conventional CMS lettuce by the PCR method, primers specific to the atp6 gene were designed on the basis of the known base sequence information (Gene Bank accession number X82387.1) (Table 2).

PCR was performed on extracted whole genome DNA as a template using a combination of primers atp6-F and atp6-R. PCR was performed with 35 cycles of heat-denaturation at 94°C for 1 minute, annealing at 60°C for 2 minutes, and elongation reaction at 72°C for 2 minutes.

PCR products were subjected to electrophoresis with 1.8% agarose gel, and the gel was soaked in an ethidium bromide solution, and then an image was taken under UV irradiation to confirm the presence of an amplified product of an expected size (209 bp). Finally, an individual in which the amplification was confirmed was selected.

In the manner described above, a CMS line "BF2MS1S" improved in low temperature growth ability was obtained.

**Table 2: Primer used for selecting cytoplasmic hybrid and base sequence thereof**

| Target gene | Primer name | Base Sequence (5'→3') | SEQ ID No. |
|---|---|---|---|
| atp6 | atp6-F | gctaactctcagtttggtcctac | 1 |
| | atp6-R | ccagaccggttaatgcaaga | 2 |

### Example 2: Detailed breeding processes of improved CMS line

The CMS line "BF2MS1S" developed in Example 1 was crossed with the lettuce with a normal cytoplasm (variety "Steady") to obtain 156 seeds of BC1 (backcrossed progeny first generation). Then, 156 BC1 seeds obtained from 1 line were sown on a 288-hole tray, and the seedlings were grown in a greenhouse in which the nighttime temperature was set to 0°C and the daytime temperature was set to 20°C. The seedlings of the BC1 exhibited good low temperature growth ability with slight variations in germination. Among the germinated individuals of the 156 BC1 seeds, 24 lines that were excellent in low temperature growth ability were selected and subjected to the backcross with Steady, thereby obtaining BC2 seeds.

Then, 20 BC2 seeds obtained from each of 24 lines were sown on 200-hole trays and the seedlings were grown in a greenhouse under the same condition. The seedlings of BC2 overall exhibited good low temperature growth ability. Although there were slight variations in germination, an improvement was made in comparison with the seedlings of BC1. Among the germinated individuals of the 480 BC2 seeds, 38 lines that were excellent in low temperature growth ability were selected and subjected to the backcross with Steady, thereby obtaining BC3 seeds.

Then, 20 BC3 seeds obtained from each of 38 lines were sown on 200-hole trays and the seedlings were grown in a greenhouse under the same condition. The seedlings of BC3 overall exhibited good low temperature growth ability, and there was no variation in germination. Among the germinated individuals of the 760 BC3 seeds, 49 lines that were excellent in low temperature growth ability were selected and subjected to the backcross with Steady, thereby obtaining BC4 seeds.

Then, 20 BC4 seeds obtained from each of 49 lines were sown on 200-hole trays and the seedlings were grown in a greenhouse under the same condition. The seedlings of BC4 overall exhibited good low temperature growth ability, and there was no variation in germination. That is, it was speculated that the cytoplasm was progressively brought into a homoplasmy state and an individual difference in the low temperature growth ability became smaller as generations passed. Among the germinated individuals of the 980 BC4 seeds, 4 lines that were excellent in low temperature growth ability were selected and subjected to the backcross with Steady, thereby obtaining BC5 seeds.

Then, 96 BC5 seeds obtained from each of 4 lines were sown on 288-hole trays and the seedlings were grown in a greenhouse under the same condition. The seedlings of BC5 exhibited good low temperature growth ability, and there was no variation in germination. Among the germinated individuals of the 384 BC5 seeds, 5 lines that were excellent in the low temperature growth ability were selected and subjected to the backcross with Steady, thereby obtaining BC6 seeds.

Then, 96 BC6 seeds obtained from each of 5 lines were sown on 288-hole trays and the seedlings were grown in a greenhouse under the same condition. The seedlings of BC6 exhibited good low temperature growth ability, and there was no variation at all in germination. It was speculated that the cytoplasm reached a homoplasmy state, thus further selection using multiple lines was determined to be unnecessary. Among the germinated individuals of the 480 BC6 seeds, one line that was excellent in low temperature growth ability was selected and subjected to the backcross with Steady, thereby obtaining BC7 seeds.

Then, 96 BC7 seeds obtained from the line were sown on 288-hole trays and the seedlings were grown in a greenhouse under the same condition. The seedlings of BC7 exhibited good low temperature growth ability, and there was no variation at all in germination. Among the germinated individuals of the 96 BC7 seeds, 1 line that was excellent in low temperature growth ability were subjected to the backcross with Steady, thereby obtaining BC8 seeds.

Then, 96 BC8 seeds obtained from the line were sown on 288-hole trays and the seedlings were grown in a greenhouse under the same condition. The seedlings of BC8 had good low temperature growth ability and did not exhibit any variation in germination, thus it was speculated that the cytoplasm was stabilized in a homoplasmy state. Among the germinated individuals of the 96 BC8 seeds, 1 that was line excellent in the low temperature growth ability was subjected to the backcross with Steady, thereby obtaining BC9 seeds.

Note that the BC10 seed of the "BF2MS1S" line, which is a progeny obtained by crossing "BF2MS1S" grown from the BC9 seed with "Steady", is internationally deposited (original deposit) at the International Patent Organism Depositary of the independent administrative corporation, National Institute of Technology and Evaluation (Room 120, 2-5-8 Kazusa Kamatari, Kisarazu, Chiba, Japan) on February 1, 2019 (identification reference given by the depositors: SSC-LET-19-001, deposition number: FERM BP-22373).

### Example 3: Confirmation of characteristics of improved CMS lettuce by germination test

In order to confirm usefulness of the improved CMS line developed in Example 1, a germination test of lettuce having various normal cytoplasms and CMS cytoplasms was performed.

Lettuce may vary in seed quality and germination capacity depending on seed production environment. Thus, the seeds were produced under the same environment for all lines used in the present examples. Furthermore, lettuce seeds may become dormant. Thus, all of the germination tests in the present examples were performed after breaking the seed dormancy by cooling cell trays after sowing at 4°C for 2 days in a refrigerator.

First, the low temperature growth ability of backcrossed progenies of the line "1216-2-T1" (deposition number: FERM BP-10421) and the line "50125-3-V1", which were the conventional CMS lettuce described in Patent Literature 1, was confirmed.

Note that "1216-2-T1" described herein is a progeny obtained by crossing a CMS line "1216-2" to "Tell me" (Sakata Seed Corp.). The seed of "1216-2-T1" has been internationally deposited at the International Patent Organism Depositary of the independent administrative corporation, National Institute of Advanced Industrial Science and Technology (Tsukuba Central 6, 1-1-1 Higashi, Tsukuba, Ibaraki, Japan) on September 29, 2005 (identification reference given by the depositors: SSC-LET-05-001, deposition number: FERM BP-10421) (Patent Literature 1).

In 128-hole cell trays, 64 seeds of "Tell me" with a normal cytoplasm and "1216-2-T1", which was the CMS lettuce described in Patent Literature 1, and 64 seeds of "V lettuce" with a normal cytoplasm and "50125-3-V1", which was the CMS lettuce described in Patent Literature 1, were sown. After sowing the cell trays were managed in a greenhouse (Kakegawa, Shizuoka, Japan) in which the nighttime temperature was set to 0°C and the daytime temperature was set to 20°C.

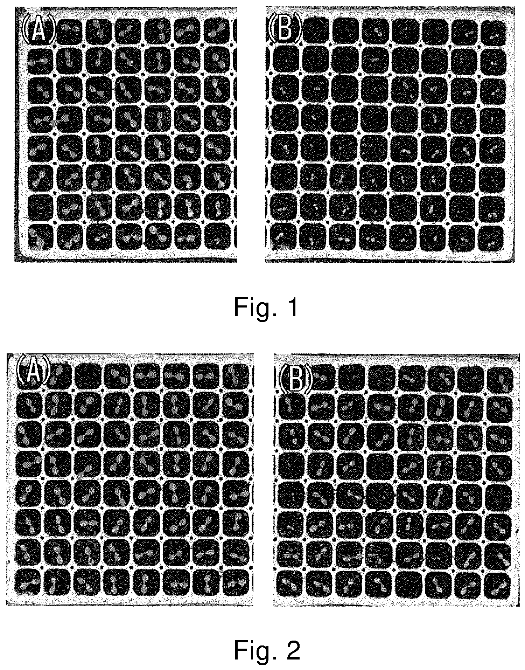

Fig. 1 shows a germination test result on Day 17 after sowing "Tell me" and "1216-2-T1".

It was confirmed that, as compared with "Tell me", "1216-2-T1" was very poor in uniformity of germination and inferior in low temperature growth ability in accordance with visual observation.

For performing quantitative evaluation, an above-ground part of the seedling in each line was cut near the ground and the weight thereof per seedling was compared.

The result was as shown in Table 3.

As shown in No. 1 and No. 2 in Table 3, the weight ratio of the weight of the above-ground part per seedling of "1216-2-T1" with respect to the weight of the above-ground part per seedling of "Tell me" was 25%.

The nuclear genome of "1216-2-T1" was identical to that of "Tell me". Thus, it was speculated that a reduction in the growth ability at low temperature in "1216-2-T1" was caused by the CMS cytoplasm.

Fig. 2 shows a germination test result on Day 17 after sowing "V lettuce" and "50125-3-V1".

It was confirmed that, as compared with "V lettuce", "50125-3-V1" was poor in uniformity of germination and inferior in the low temperature growth ability in accordance with visual observation.

For performing quantitative evaluation, an above-ground part of the seedling in each line was cut near the ground and the weight thereof per seedling was compared.

The result was as shown in Table 3.

As shown in No. 3 and No. 4 in Table 3, the weight ratio of the weight of the above-ground part per seedling of "50125-3-V1" with respect to the weight of the above-ground part per seedling of "V lettuce" was 55%.

The nuclear genome of "50125-3-V1" was identical to that of "V lettuce". Thus, it was speculated that a reduction in the growth ability at the low temperature in "50125-3-V1" was caused by the conventional CMS cytoplasm.

As described above, the germination test in the greenhouse under low temperature condition confirmed that the conventional CMS lettuce had reduced low temperature growth ability.

However, it was speculated that the test in the greenhouse was easily affected by the natural environment. Thus, the same germination test was performed in an artificial environment by using an artificial climate chamber.

First, the germination test of "50125-3-V1", which was the conventional CMS lettuce described in Patent Literature 1, was performed under the optimal growth temperature condition by using an artificial climate chamber. The artificial climate chamber was set such that the temperature was held at 20°C, which was the optimal germination and growth temperature for lettuce, with 12-hour illumination.

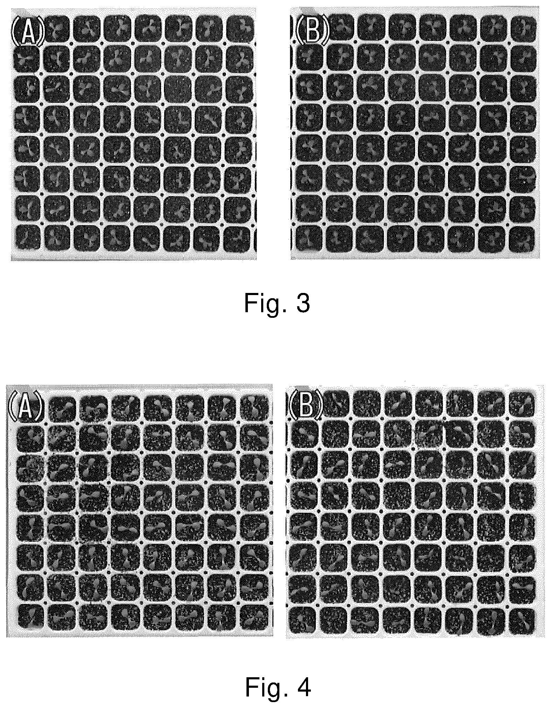

Fig. 3 shows a germination test result on Day 16 after sowing "V lettuce" and "50125-3-V1" when the artificial climate chamber was set to the optimal growth temperature condition.

Under the optimal temperature condition, "50125-3-V1" exhibited the growth ability fully comparable to that of "V lettuce" in accordance with visual observation.

For performing quantitative evaluation, an above-ground part of the seedling in each line was cut near the ground and the weight thereof per seedling was compared.

The result was as shown in Table 3.

As shown in No. 5 and No. 6 in Table 3, the weight ratio of the weight of the above-ground part per seedling of "50125-3-V1" with respect to the weight of the above-ground part per seedling of "V lettuce" was 116%. Under the optimal temperature condition, the growth ability of "50125-3-V1" slightly exceeded that of "V lettuce" with a normal cytoplasm.

Next, the germination test was performed under a low temperature condition artificially created by using the artificial climate chamber.

The artificial climate chamber was set such that the nighttime temperature was 5°C and the daytime temperature was 20°C with 12-hour illumination. Due to the performance limit of the artificial climate chamber, the nighttime temperature could not be set to 0°C, thus it was set to the lower limit of 5°C.

Fig. 4 shows a germination test result on Day 17 after sowing "V lettuce" and "50125-3-V1" when the artificial climate chamber was set to the low temperature condition.

It was confirmed that, as compared with "V lettuce", "50125-3-V1" was poor in uniformity of germination and inferior in low temperature growth ability, in accordance with visual observation.

For performing quantitative evaluation, an above-ground part of the seedling in each line was cut near the ground and the weight thereof per seedling was compared.

The result was as shown in Table 3.

As shown in No. 7 and No. 8 in Table 3, the weight ratio of the weight of the above-ground part per seedling of "50125-3-V1" with respect to the weight of the above-ground part per seedling of "V lettuce" was 66%. It could be confirmed that the reduction in growth ability at the low temperature in "50125-3-V1" was reproducible under the low temperature condition created by the artificial climate chamber. Further, in the test using the artificial climate chamber, the daytime temperature was the optimal growth temperature of 20°C. Thus, it was found that the reduction in growth ability due to low temperature was solely caused by the nighttime temperature under the low temperature condition.

As described above, it was found that the CMS lettuce disclosed in Patent Literature 1 exhibited the higher growth ability than "V lettuce" with a normal cytoplasm under the optimal temperature condition, but it had the reduced growth ability under the condition in which the nighttime temperature was low. That is, no attention was paid on the reduction in the growth ability of the prior CMS plant of the genus Lactuca under the low temperature condition, and this problem was not known at the time of filing Patent Literature 1. Needless to say, in Patent Literature 1, there is no description nor suggestion about this problem.

Next, an improved CMS line "BF2MS4V" was obtained in the same manner as in Example 1 except that "V lettuce" was used as the plant of the genus Lactuca with a normal cytoplasm.

"BF2MS4V" was obtained by using "50125-3-V1" as the cytoplasm donor parent and "V lettuce" as the cytoplasm acceptor parent when the asymmetric protoplast fusion was performed.

"BF2MS4V", which is an improved CMS line developed in accordance with Example 1, is developed by the asymmetric protoplast fusion using "50125-3-V1" as the cytoplasm donor parent, thus it has the same nuclear genome as that of "V lettuce".

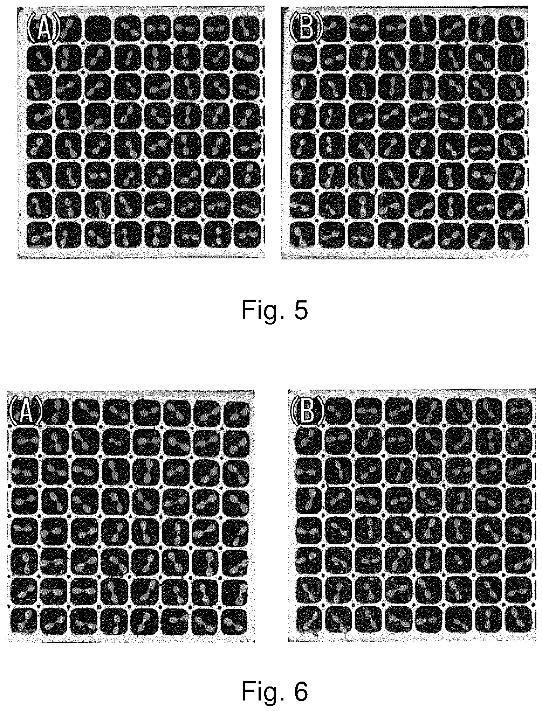

Fig. 5 shows a germination test result on Day 17 after sowing "V lettuce" and "BF2MS4V" under a greenhouse environment in which a nighttime temperature is set to 0°C and a daytime temperature is set to 20°C.

"BF2MS4V" exhibited the uniform germination and low temperature growth ability comparable to those of "V lettuce" in accordance with visual observation.

For performing quantitative evaluation, an above-ground part of the seedling in each line was cut near the ground and the weight thereof per seedling was compared.

The result was as shown in Table 3.

As shown in No. 3 and No. 9 in Table 3, the weight ratio of the weight of the above-ground part per seedling of "BF2MS4V" with respect to the weight of the above-ground part per seedling of "V lettuce" was 90%. The weight of the above-ground part per seedling of "BF2MS4V" under the low temperature condition was inferior by about 10% to that of the plant with a normal cytoplasm. However, on the basis of the comparison between No. 4 and No. 9 in Table 3, the weight ratio of the above-ground part per seedling of "BF2MS4V" with respect to that of "50125-3-V1", which was the CMS lettuce in Patent Literature 1, improved to 165%.

From this result, it was found that, in the CMS lettuce associated with an unfavorable trait such as a reduction in growth ability due to low temperature, the CMS cytoplasm could be improved by the recurrent asymmetric protoplast fusion with lettuce with a normal cytoplasm.

Next, an improved CMS line "BF2MS2M" was obtained in the same manner as in Example 1 except that a breeding line "M8-039" (Sakata Seed Corp.) was used as the plant of the genus Lactuca with a normal cytoplasm.

"BF2MS2M" was obtained by using "50125-3-V1" as the cytoplasm donor parent and "M8-039" as the cytoplasm acceptor parent when the asymmetric protoplast fusion was performed.

Fig. 6 shows a germination test result on Day 17 after sowing the breeding lines "M8-039" and "BF2MS2M" under a greenhouse environment in which the nighttime temperature was set to 0°C and the daytime temperature was set to 20°C.

As shown in the figure, "BF2MS2M" exhibited the low temperature growth ability comparable to that of "M8-039" in accordance with visual observation.

For performing quantitative evaluation, an above-ground part of the seedling in each line was cut near the ground and the weight thereof per seedling was compared.

The result was as shown in Table 3.

As shown in No. 10 and No. 11 in Table 3, the weight ratio of the weight of the above-ground part per seedling in "BF2MS2M" with respect to the weight of the above-ground part per seedling of "M8-039" was 93%. The difference in the weight of the above-ground part per seedling compared to that of the plant with a normal cytoplasm showed only a small reduction of 7%, inferring that the CMS cytoplasm was significantly improved as compared with the CMS lettuce in Patent Literature 1, namely, "1216-2-T1" with a reduction of 75% and "50125-3-V1" with a reduction of 45%.

Thus, it was confirmed that the cytoplasm could be improved by the asymmetric back fusion with different nuclear genome.

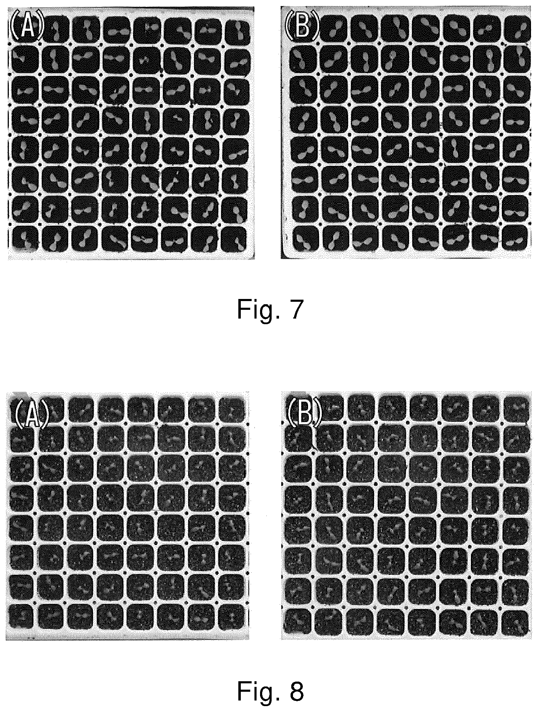

Next, Fig. 7 shows a germination test result on Day 17 after sowing "Steady" and "BF2MS1S" under a greenhouse environment in which a nighttime temperature is set to 0°C and a daytime temperature is set to 20°C.

As described in detail in Example 1, in the breeding processes of "BF2MS1S", "BF2MS1S" was developed by the asymmetric protoplast fusion using "50125-3-V1" as the cytoplasm donor parent and "Steady" as the cytoplasm acceptor parent.

It was observed that "BF2MS1S" was superior in the uniformity of germination to "Steady" and had comparable low temperature growth ability in accordance with visual observation.

For performing quantitative evaluation, an above-ground part of the seedling in each line was cut near the ground and the weight thereof per seedling was compared.

The result was as shown in Table 3.

As shown in No. 12 and No. 13 in Table 3, the weight ratio of the weight of the above-ground part per seedling of "BF2MS1S" with respect to the weight of the above-ground part per seedling of "Steady" was 101%.

Further, in order to confirm the characteristics of the cytoplasm of "BF2MS1S" under the artificial environment, the germination test was performed using the artificial climate chamber. The artificial climate chamber was set such that the nighttime temperature was 5°C and the daytime temperature was 20°C with 12-hour illumination. The nighttime temperature was not set to 0°C as in the greenhouse, because the artificial climate chamber had the lower limit of setting temperature of 5°C.

Fig. 8 shows a germination test result on Day 17 after sowing "Steady" and "BF2MS1S" using the artificial climate chamber.

The low temperature growth ability of the "Steady" and "BF2MS1S" was comparable to each other in accordance with visual observation.

For performing quantitative evaluation, an above-ground part of the seedling in each line was cut near the ground and the weight thereof per seedling was compared.

The result was as shown in Table 3.

As shown in No. 14 and No. 15 in Table 3, the weight ratio of the weight of the above-ground part per seedling of "BF2MS1S" with respect to the weight of "Steady" was 106%, indicating that the low temperature growth ability of "BF2MS1S" was comparable to or better than that of "Steady" with a normal cytoplasm also under the artificial environment.

"BF2MS1S" had identical nuclear genome to that of "Steady" with a normal cytoplasm. Thus, it could be confirmed that the cytoplasm of "BF2MS1S" had ability comparable to or better than that of the normal cytoplasm in terms ofto low temperature growth.

As described in Example 2, BC7 of "BF2MS1S" was bred by performing the recurrent backcross.

BC7 of "BF2MS1S" was transplanted to a farm field (Azumino, Nagano, Japan) to examine its traits in the head-forming period.

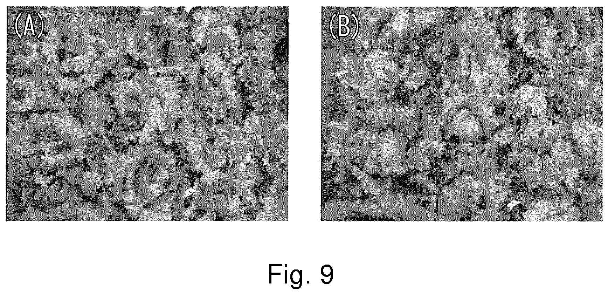

The result was as shown in Fig. 9.

"BF2MS1S" showed no morphological difference from "Steady" with a normal cytoplasm, confirming that "BF2MS1S" had the very excellent CMS. Further, "BF2MS1S" did not produce any individual causing fertility restoration until reaching BC7, confirming that "BF2MS1S" had very stable CMS characteristics.

**Table 3: Effects by various prior CMS cytoplasm under the low temperature condition**

| No. | Line name | Cytoplasm | Number of planted seeds | Number of germinated seeds | Germination rate | Condition | A | B (g/seedling) | Weight comparison of above-ground part | |
|---|---|---|---|---|---|---|---|---|---|---|
| | | | | | | | | | C | D |
| 1 | Tell me | N | 64 | 62 | 97% | a | 23 | 0.0308 | 1 | 100% |
| 2 | 1216-2-T1 | S | 64 | 39 | 61% | a | 15 | 0.0076 | 1 | 25% |
| 3 | V lettuce | N | 64 | 63 | 98% | a | 24 | 0.0253 | 3 | 100% |
| 4 | 50125-3-V1 | S | 64 | 59 | 92% | a | 24 | 0.0138 | 3 | 55% |
| 5 | V lettuce | N | 64 | 62 | 97% | b | 23 | 0.0250 | 5 | 100% |
| 6 | 50125-3-V1 | S | 64 | 64 | 100% | b | 24 | 0.0290 | 5 | 116% |
| 7 | V lettuce | N | 64 | 63 | 98% | c | 23 | 0.0289 | 7 | 100% |
| 8 | 50125-3-V1 | S | 64 | 59 | 92% | c | 22 | 0.0191 | 7 | 66% |
| 9 | BF2MS4V | S | 64 | 64 | 100% | a | 24 | 0.0228 | 3 | 90% |
| 10 | M8-039 | N | 64 | 64 | 100% | a | 24 | 0.0308 | 10 | 100% |
| 11 | BF2MS2M | S | 64 | 64 | 100% | a | 24 | 0.0285 | 10 | 93% |
| 12 | Steady | N | 64 | 64 | 100% | a | 24 | 0.0273 | 12 | 100% |
| 13 | BF2MS1S | S | 64 | 64 | 100% | a | 24 | 0.0277 | 12 | 101% |
| 14 | Steady | N | 64 | 62 | 97% | c | 23 | 0.0310 | 14 | 100% |
| 15 | BF2MS1S | S | 64 | 64 | 100% | c | 24 | 0.0328 | 14 | 106% |

| | | | | | | | | | | |
|---|---|---|---|---|---|---|---|---|---|---|
| A: Number of individuals for weight measurement (three columns of cell tray) B: Average weight of above-ground part of seedling per seedling (g/seedling) C: Control No. used in comparison of weight of above-ground part D: Ratio of average weight of above-ground part of seedling with CMS cytoplasm when average weight of above-ground part of corresponding seedling with normal cytoplasm is defined as 100% N: Normal cytoplasm S: Male sterile cytoplasm a: Performed in greenhouse (setting: nighttime temperature 0°C, daytime temperature 20°C) b: Artificial climate chamber (nighttime temperature 20°C, daytime temperature 20°C, 12-hour illumination) c: Artificial climate chamber (nighttime temperature 5°C, daytime temperature 20°C, 12-hour illumination) | | | | | | | | | | |

### Example 4

In order to analyze the plasmatype of the improved CMS lettuce "BF2MS1S" developed in Example 1, 44 kinds of primer sets shown in Table 4 were designed on the basis of the known sunflower mitochondrial genome base sequence information (Gene Bank accession number KF815390).

In a case where an intergenic region can be amplified, the primers were preferentially designed so as to target such a region as a marker, while, in other regions, the primers were designed such that a physical distance between markers has an interval of from 5,000 to 10,000 bp.

As sample materials, "V lettuce" with a normal cytoplasm, CMS sunflower "IB5", the CMS lettuce "1216-2-T1" and "50125-3-V1" described in Patent Literature 1, and the improved CMS lettuce "BF2MS1S" developed in the present invention were used.

PCR was performed using the primer sets described in Table 4 with whole genome DNA extracted from each sample material as a template. PCR was performed with 35 cycles of heat-denaturation at 94°C for 1 minute, annealing at 60°C for 2 minutes, and elongation reaction at 72°C for 2 minutes.

In the case where a polymorphism was not detected between "V lettuce" with a normal cytoplasm and the CMS sunflower "IB5", the PCR product was treated by the restriction enzyme described in Table 4 in order to perform PCR-RFLP. These PCR products were subjected to electrophoresis with 1.8% agarose gel. Then, the gel was soaked in an ethidium bromide solution and an image was taken under UV irradiation to examine the presence of the PCR products and the polymorphisms.

The analysis results of the cytoplasm using the PCR method and the PCR-RFLP method were shown in Table 5. In Table 5, "Ha" represents an H. annuus type, "Ls" represents an L. sativa type, and "Hetero" represents a heterozygous type of these two. "0" represents no detection of the corresponding marker.

Using 44 kinds of the primer sets, the polymorphisms of sunflower type were detected with 24 sets (including hetero types) in "1216-2-T1", 10 sets in "50125-3-V1", and 3 sets in "BF2MS1S".

This result confirmed that the ratio of the mitochondrial genome regions derived from sunflower in the CMS lettuce decreased each time the back fusion was performed by the asymmetric protoplast fusion, and a majority of the mitochondrial genome in "BF2MS1S" was replaced with the mitochondrial genome derived from lettuce.

Based on the above, it was speculated that, in the improvement in low temperature growth ability of "BF2MS1S", while the genome region derived from sunflower involved in CMS was preserved, a majority of other mitochondrial genome regions were replaced with the lettuce type by the back fusion, using the asymmetric protoplast fusion, which resolved the incompatibility between the nuclear genome and the mitochondrial genome and made it possible to improve low temperature growth ability while maintaining the trait of CMS.

Note that the result in Table 5 merely showed one example of the analysis result of the individuals exhibiting the cytoplasmic male sterility, and it does not necessarily mean that the CMS lettuce improved in the low temperature growth ability always has such a structure of the mitochondrial genome.

**Table 4: Primer used for analyzing cytoplasm and base sequence thereof**

| Primer No | Primer name | Base Sequence (5'→3') | SEQ ID No. | Restriction enzyme |
|---|---|---|---|---|
| 1 | SHaMt-1Kb-1F | agccagtaacagacacgacaga | 3 | - |
| | SHaMt-1Kb-2R | cgaaccgtccaatgaagaataa | 4 | |
| 2 | SHaMt-6Kb-1F | tctgtcgcttttaaccactcg | 5 | TaqI |
| | SHaMt-6Kb-2R | ttcaaatcctacttggggagaa | 6 | |
| 3 | SHaMt-10Kb-1F | tcaattagggaatagcgcaga | 7 | - |
| | SHaMt-10Kb-2R | tggtgtgctaaccaaagaacc | 8 | |
| 4 | SHaMt-16Kb-1F | agatcggatttccttttcgtg | 9 | - |
| | SHaMt-16Kb-2R | agcctatcgtctcagcctttc | 10 | |
| 5 | SHaMt-24Kb-1F | gcgctaggcacctcttactct | 11 | - |
| | SHaMt-24Kb-2R | ttgacagattcctggtcgaag | 12 | |
| 6 | SHaMt-30Kb-1F | gacagcaaaagcagcgattag | 13 | - |
| | SHaMt-30Kb-2R | atatgggacaagcggtaggtt | 14 | |
| 7 | SHa Mt-36Kb-1F | tgcaatgatggagatggactac | 15 | DraI |
| | SHaMt-36Kb-2R | aacctccgaaacgataaacaga | 16 | |
| 8 | SHaMt-45Kb-1F | tggttctcatttcctggtttg | 17 | - |
| | SHaMt-45Kb-2R | caaagtgttctcggactgcat | 18 | |
| 9 | SHaMt-54Kb-1F | gtgaggctccactctatcctgt | 19 | - |
| | SHaMt-54Kb-2R | caatgctaaatgtccagggagt | 20 | |
| 10 | SHaMt-60Kb-1F | gtactcaacaggaagcgtcaca | 21 | - |
| | SHaMt-60Kb-2R | cgaaagcggatcagtacaaac | 22 | |
| 11 | SHaMt-66Kb-1F | atcacgagtttttctccattcc | 23 | DdeI |
| | SHaMt-66Kb-2R | tttcccggtagaaccctatttc | 24 | |
| 12 | SHaMt-72Kb-1F | tagacagcaagcaagcgaga | 25 | - |
| | SHaMt-72Kb-2R | gccctacctgatccatttca | 26 | |
| 13 | SHaMt-77Kb-1F | ctgccgatgtagctcttgttc | 27 | DdeI |
| | SHaMt-77Kb-2R | ggatgcacgtcttttgttcat | 28 | |
| 14 | SHaMt-82Kb-1F | caaaggcgagcatatccatt | 29 | - |
| | SHaMt-82Kb-2R | tccatttccctctcggtactt | 30 | |
| 15 | SHaMt-87Kb-1F | ccttgatccacttggctacatc | 31 | HinfI |
| | SHaMt-87Kb-2R | ggaatcgaacccgtgtctt | 32 | |

**Table 4-2**

| Primer No | Primer name | Base Sequence (5'→3') | SEQ ID No. | Restriction enzyme |
|---|---|---|---|---|
| 16 | SHaMt-93Kb-1F | aaagagtccagccgagcata | 33 | MboI |
| | SHaMt-93Kb-2R | aatcactatggaaccggacct | 34 | |
| 17 | SHaMt-98Kb-1F | taagagcaaacaaggcaatgg | 35 | - |
| | SHaMt-98Kb-2R | cggattcacctaaacatcttcc | 36 | |
| 18 | SHaMt-104Kb-1F | tagcttggtaatccctcgcttt | 37 | - |
| | SHaMt-104Kb-2R | aagttgatgattcccaccaaga | 3 | |
| 19 | SHaMt-112Kb-1F | aatcccaggagctaatccaaa | 39 | HinfI |
| | SHaMt-112Kb-2R | atgatggcctttctgatctcat | 40 | |
| 20 | SHaMt-120Kb-1F | ccattgtagttggagcgagag | 41 | - |
| | SHaMt-120Kb-2R | ttccctgttccaaacatttcc | 42 | |
| 21 | SHaMt-126Kb-1F | ttgttgagaggacttgcatcc | 43 | - |
| | SHaMt-126Kb-2R | cataccttggcagtgtggtct | 44 | |
| 22 | SHaMt-133Kb-1F | tcttggagacagggcaataga | 45 | HpaII |
| | SHaMt-133Kb-2R | catcttaggcggtaagcgaag | 46 | |
| 23 | SHaMt-142Kb-1F | gaaccgggcgtactacttctc | 47 | - |
| | SHaMt-142Kb-2R | caaataaggttacgggcttcc | 48 | |
| 24 | SHaMt-147Kb-1F | taccctgtaagcgtgaagtcag | 49 | - |
| | SHaMt-147Kb-2R | atgaaccactccggtacaaca | 50 | |
| 25 | SHaMt-152Kb-1F | agcggagcctagaagaagatg | 51 | - |
| | SHaMt-152Kb-2R | tgaggaaggtacagcccaac | 52 | |
| 26 | SHaMt-160Kb-1F | gaaagcaacttcaccgaaagg | 53 | - |
| | SHaMt-160Kb-2R | atttcccacgtatggagcact | 54 | |
| 27 | SHaMt-169Kb-1F | cgtacaaccatcaaaccagaga | 55 | - |
| | SHaMt-169Kb-2R | aacttgaaggtatggctgagtg | 56 | |
| 28 | SHaMt-176Kb-1F | gtcgatcttgtaacccacgaa | 57 | - |
| | SHaMt-176Kb-2R | gtcactcactggaggtttgga | 58 | |
| 29 | SHaMt-183Kb-1F | tcaagtgaagtgcaagatcca | 59 | - |
| | SHaMt-183Kb-2R | gcgttttgtcccctatctctc | 60 | |
| 30 | SHaMt-191Kb-1F | ccaggggacaaatcaatagga | 61 | - |
| | SHaMt-191Kb-2R | gaataacatggcctgaaacga | 62 | |

**Table 4-3**

| Primer No | Primer name | Base Sequence (5'→3') | SEQ ID No. | Restriction enzyme |
|---|---|---|---|---|
| 31 | SHaMt-196Kb-1F | gcaccaaaccaatccatcttc | 63 | - |
| | SHaMt-196Kb-2R | tggcttcctccctagctctac | 64 | |
| 32 | SHaMt-201Kb-1F | gagcttctcccaaggatgtct | 65 | - |
| | SHaMt-201Kb-2R | attctgtgatcgaatgccact | 66 | |
| 33 | SHaMt-208Kb-1F | aaccgcttcactacttccaaga | 67 | - |
| | SHaMt-208Kb-2R | cgaaataagtcgggtagtgc | 68 | |
| 34 | SHaMt-214Kb-1F | cacttgcggttaattcgttgt | 69 | HinfI |
| | SHaMt-214Kb-2R | ctttgatggatgccctttgta | 70 | |
| 35 | SHa Mt-221 Kb-1F | tagtcgtcatgggaaagaaagg | 71 | - |
| | SHaMt-221Kb-2R | cctatatatacgggtccgtgcag | 72 | |
| 36 | SHaMt-230Kb-1F | taatcgccgaaattgtacgac | 73 | - |
| | SHaMt-230Kb-2R | gacatcacaagggttggtttg | 74 | |
| 37 | SHaMt-238Kb-1F | cca cttcg a ctttca gtctcg | 75 | - |
| | SHaMt-238Kb-2R | tgggttggacgacatacatct | 76 | |
| 38 | SHaMt-244Kb-1F | taatggttggagcagcaaact | 77 | - |
| | SHaMt-244Kb-2R | cacgataaattcagcactggtc | 78 | |
| 39 | SHaMt-251Kb-1F | aggttccccactcttctttga | 79 | TaqI |
| | SHaMt-251Kb-2R | gaagcaaatggaaaaggaacac | 80 | |
| 40 | SHaMt-260Kb-1F | agtgcagacgaagtaacacgaa | 81 | RsaI |
| | SHaMt-260Kb-2R | tcacgctctgtaggatttgaac | 82 | |
| 41 | SHaMt-267Kb-1F | ctctctcccg a tg a ctg a ctct | 83 | - |
| | SHaMt-267Kb-2R | caggttcagcacgaaatcataa | 84 | |
| 42 | SHaMt-275Kb-1F | gaattgttctcaagcgaatgg | 85 | - |
| | SHaMt-275Kb-2R | tttcgtgagtatgcggttctt | 86 | |
| 43 | SHaMt-284Kb-1F | aggcatggaaggaatacgact | 87 | - |
| | SHaMt-284Kb-2R | gccttgctctaaaggagcttg | 88 | |
| 44 | SHaMt-293Kb-1F | aagtggtgaggtttgcagaga | 89 | - |
| | SHaMt-293Kb-2R | ggccagagctacttgggtttag | 90 | |

**Table 5: Analysis result of the cytoplasm of CMS plant of the genus Lactuca by a PCR and a PCR-RFLP method**

| **Primer No.** | **CMS sunflower "IB5"** | **Lettuce "V Lettuce"** | **1216-2-T1** | **50125-3-V1** | **BF2MS1S** |
|---|---|---|---|---|---|
| 1 | Ha | 0 | 0 | 0 | 0 |
| 2 | Ha | Ls | Ls | Ls | Ls |
| 3 | Ha | 0 | 0 | 0 | 0 |
| 4 | Ha | 0 | Ha | Ha | Ha |
| 5 | Ha | 0 | Ha | Ha | Ha |
| 6 | Ha | 0 | Ha | Ha | Ha |
| 7 | Ha | Ls | Ha | Ls | Ls |
| 8 | Ha | 0 | Ha | 0 | 0 |
| 9 | Ha | 0 | Ha | 0 | 0 |
| 10 | Ha | 0 | Ha | 0 | 0 |
| 11 | Ha | Ls | Ls | Ls | Ls |
| 12 | Ha | 0 | 0 | 0 | 0 |
| 13 | Ha | Ls | Ls | Ls | Ls |
| 14 | Ha | 0 | 0 | 0 | 0 |
| 15 | Ha | Ls | Ls | Ls | Ls |
| 16 | Ha | Ls | Ls | Ls | Ls |
| 17 | Ha | 0 | Ha | Ha | 0 |
| 18 | Ha | 0 | Ha | Ha | 0 |
| 19 | Ha | Ls | Ha | Ha | Ls |
| 20 | Ha | 0 | Ha | 0 | 0 |
| 21 | Ha | 0 | 0 | 0 | 0 |
| 22 | Ha | Ls | Ls | Ls | Ls |
| 23 | Ha | 0 | 0 | 0 | 0 |
| 24 | Ha | 0 | 0 | 0 | 0 |
| 25 | Ha | 0 | 0 | 0 | 0 |
| 26 | Ha | 0 | Ha | 0 | 0 |
| 27 | Ha | 0 | 0 | 0 | 0 |
| 28 | Ha | Ls | Ha | Ls | Ls |
| 29 | Ha | 0 | Ha | Ha | 0 |
| 30 | Ha | 0 | 0 | 0 | 0 |
| 31 | Ha | 0 | 0 | 0 | 0 |
| 32 | Ha | 0 | Ha | Ha | 0 |
| 33 | Ha | 0 | 0 | 0 | 0 |
| 34 | Ha | Ls | Ha | Ls | Ls |
| 35 | Ha( | 0 | 0 | 0 | 0 |
| 36 | Ha | 0 | Ha | 0 | 0 |
| 37 | Ha | 0 | Ha | 0 | 0 |
| 38 | Ha | 0 | 0 | 0 | 0 |
| 39 | Ha | Ls | Ha | Ha | Ls |
| 40 | Ha | Ls | Hete ro | Ls | Ls |
| 41 | Ha | 0 | Ha | 0 | 0 |
| 42 | Ha | 0 | Ha | Ha | 0 |
| 43 | Ha | 0 | Ha | 0 | 0 |
| 44 | Ha | 0 | Ha | 0 | 0 |

### (Description of symbols in Tables)

- Ha:: H. annuus type
- Ls:: L. sativa type
- Hetero:: hetero type of these two
- 0:: no detection of corresponding marker

## Claims

1. A cytoplasmic male sterile plant of the genus Lactuca having low temperature growth ability comparable to that of a plant of the genus Lactuca with a normal cytoplasm, or a progeny thereof.

2. A cytoplasmic male sterile plant of the genus Lactuca or the progeny thereof according to claim 1, including DNA derived from the mitochondrial genome of a plant of the genus Helianthus in the mitochondrial genome.

3. A cytoplasmic male sterile plant of the genus Lactuca or the progeny thereof according to claim 1 or 2, being obtained by performing asymmetric protoplast fusion multiple times using the plant of the genus Lactuca with the normal cytoplasm as a cytoplasm acceptor parent.

4. A cytoplasmic male sterile plant of the genus Lactuca or the progeny thereof according to any one of claims 1 to 3, being obtained by performing the asymmetric protoplast fusion using an existing-cytoplasmic-male-sterile-plant of the genus Lactuca as a cytoplasm donor parent and the plant of the genus Lactuca with the normal cytoplasm as the cytoplasm acceptor parent.

5. A cytoplasmic male sterile plant of the genus Lactuca including DNA derived from the mitochondrial genome of a plant of the genus Helianthus in the mitochondrial genome, or a progeny thereof, wherein the cytoplasmic male sterile plant of the genus Lactuca or the progeny thereof is obtained by:
performing asymmetric protoplast fusion multiple times using the plant of the genus Lactuca with a normal cytoplasm as a cytoplasm acceptor parent; or
performing the asymmetric protoplast fusion using the existing-cytoplasmic-male-sterile-plant of the genus Lactuca as a cytoplasm donor parent and the plant of the genus Lactuca with the normal cytoplasm as the cytoplasm acceptor parent.

6. A cytoplasmic male sterile plant of the genus Lactuca or the progeny thereof according to any one of claims 1 to 5, wherein the cytoplasmic male sterile plant of the genus Lactuca is derived from Lactuca sativa L. or an interspecific hybrid plant of the plant of the genus Lactuca.

7. A cytoplasmic male sterile plant of the genus Lactuca including mitochondrial genome of a plant which is specified by a deposition number FERM BP-22373, or a progeny thereof.

8. A cytoplasmic male sterile plant of the genus Lactuca which is specified by a deposition number FERM BP-22373, or a progeny thereof.

9. A part of a plant body of the cytoplasmic male sterile plant of the genus Lactuca or the progeny thereof according to any one of claims 1 to 8.

10. A seed of the cytoplasmic male sterile plant of the genus Lactuca or the progeny thereof according to any one of claims 1 to 8.

11. Mitochondrial genome included in the cytoplasmic male sterile plant of the genus Lactuca or the progeny thereof according to any one of claims 1 to 8, the part of the plant body according to claim 9, or the seed according to claim 10.

12. An asymmetric back-fusion method of a plant, comprising performing asymmetric protoplast fusion once or multiple times using: a cytoplasmic male sterile plant of the genus Lactuca as a cytoplasm donor parent, the cytoplasmic male sterile plant of the genus Lactuca being a plant obtained by the asymmetric protoplast fusion or a progeny thereof; and
a plant of the genus Lactuca with a normal cytoplasm as a cytoplasm acceptor parent, the plant of the genus Lactuca with the normal cytoplasm being one of the plants used in the initial asymmetric protoplast fusion.

13. A method for improving the mitochondrial genome in a cytoplasm of a plant of the genus Lactuca using the method according to claim 12.

14. A method for producing a cytoplasmic male sterile plant of the genus Lactuca having low temperature growth ability comparable to that of a plant of the genus Lactuca with a normal cytoplasm, or a progeny thereof, the method comprising performing asymmetric protoplast fusion using the existing-cytoplasmic-male-sterile-plant of the genus Lactuca as a cytoplasm donor parent and the plant of the genus Lactuca with the normal cytoplasm as a cytoplasm acceptor parent.

15. The method according to claim 14, wherein the cytoplasmic male sterile plant of the genus Lactuca is Lactuca sativa L. or a plant derived from an interspecific hybrid plant of the plant of the genus Lactuca.

16. A method for producing a first filial hybrid seed, comprising crossing the cytoplasmic male sterile plant of the genus Lactuca or the progeny thereof according to any one of claims 1 to 8 as a seed parent with a plant of the genus Lactuca crossable with the former plant as a pollen parent and producing the first filial hybrid seed from the seed parent after the crossing.

17. The method according to claim 16, wherein the pollen parent is Lactuca sativa L. or an interspecific hybrid plant of the plant of the genus Lactuca.

18. A first filial hybrid seed developed by the method according to claim 16 or 17, or a first filial hybrid plant grown from the seed, or a progeny thereof, or a part of a plant body thereof.

19. A method for producing a plant of the genus Lactuca having a favorable trait and exhibiting cytoplasmic male sterility, the method comprising performing recurrent backcross between the cytoplasmic male sterile plant of the genus Lactuca or the progeny thereof according to any one of claims 1 to 8 and a plant of the genus Lactuca having the favorable trait for cytoplasmic replacement.

20. The method according to claim 19, wherein the plant of the genus Lactuca having the favorable trait is derived from Lactuca sativa L.
